(19) Europäisches Patentamt
European Patent Office
Office européen des brevets

(11) Numéro de publication: **0 041 935**
**B1**

(12) **FASCICULE DE BREVET EUROPEEN**

(45) Date de publication du fascicule du brevet: **19.09.84**

(51) Int. Cl.³: **C 07 D 519/04,** A 61 K 31/395

(21) Numéro de dépôt: **81870028.8**

(22) Date de dépôt: **09.06.81**

(54) **Dérivés n-(vinblastinoyl-23) d'acides aminés et de peptides ainsi que leur préparation.**

(30) Priorité: **10.06.80 LU 82514**
**23.12.80 LU 83034**

(43) Date de publication de la demande:
**16.12.81 Bulletin 81/50**

(45) Mention de la délivrance du brevet:
**19.09.84 Bulletin 84/38**

(84) Etats contractants désignés:
**AT BE CH DE FR GB IT LI LU NL SE**

(56) Documents cités:
**BE-A- 813 168**

**JOURNAL OF MEDICINAL CHEMISTRY, 1979, vol. 22, no. 4 Washington, D.C. US R.A. CONRAD et al.: "Structure-activity relationships of dimeric catharanthus alkaloids.2.Experimental antitumor activities of N-substituted deacetylvinblastine amide (vindesine) sulfates 1-3", pages 391-400**

(73) Titulaire: **OMNICHEM Société anonyme**
**12 Avenue de Broqueville**
**B-1150 Bruxelles (BE)**

(72) Inventeur: **Trouet, André Benoit Léon**
**29 Predikherenberg**
**B-3900 Winksele (BE)**
Inventeur: **Rao, Kandukuri Sivaprasada Bushana**
**11 rue Kwakambienne**
**B-1331 Rosières (BE)**
Inventeur: **Hannart, Jean Alfred Alphonse**
**25 avenue d'El Pirere**
**B-1302 Dion Valmont (BE)**

(74) Mandataire: **Van Malderen, Michel**
**p.a. Freylinger & Associés 22 avenue J.S. Bach (bte 43)**
**B-1080 Bruxelles (BE)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**Description**

La présente invention se rapporte à de nouveaux alcaloïdes bis-indoliques. Plus particulièrement, l'invention est relative à de nouveaux dérivés de couplage d'acides aminés avec la vinblastine, y compris des dérivés peptidiques, à leur procédé de préparation, à leur application en tant qu'agents antitumoraux et aux compositions pharmaceutiques les contenant.

Ces nouveaux dérivés de la vinblastine peuvent être utilisés pour le traitement des leucémies et des tumeurs malignes chez l'homme.

Les alcaloïdes bisindoliques du type de la vinblastine sont des composés bien connus possédant le squelette carboné de la formule générale suivante:

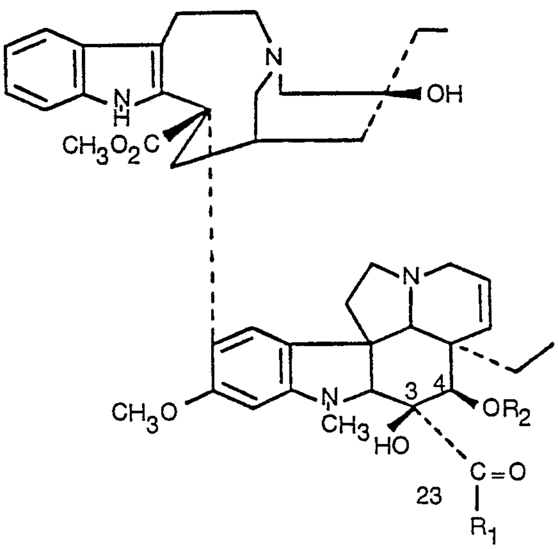

(I)

On citera à titre d'exemple la vincaleukoblastine (brevet américain 3 097 137), la leurocristine ou vincristine et la leurosidine (brevet américain 3 205 220), le vingylcinate (brevet belge 659 112) et la vindésine (brevet belge 813 168). Ce dernier composé a été obtenu par transformation chimique de la vinblastine naturelle (I, $R_1$=$OCH_3$, $R_2$=$COCH_3$), qui elle-même est obtenue par extraction à partir de feuilles de *Catharanthus roseus*.

La vinblastine, la vincristine et la vindésine sont commercialisées pour leur utilisation en thérapeutique humaine, plus particulièrement pour le traitement de leucémies et de certaines tumeurs solides.

Ces médicaments possèdent néanmoins des effets secondaires défavorables. La vincristine est neurotoxique et la vinblastine est fortement toxique au niveau des tissus hématopoétiques.

Leur mécanisme d'action est analogue à celui qui a été postulé pour l'action antimitotique de la colchicine. Ces médicaments agiraient par inhibition de la polymérisation de la tubuline en microtubules et arrêt subséquent de la division cellulaire en métaphase.

L'utilisation de complexes 1:1 d'alcaloïdes bisindoliques antitumoraux avec la tubuline a été décrite dans le brevet belge 854 053.

Dans certains cas, il peut en résulter une toxicité moindre et une activité chimiothérapeutique plus importante que celles des alcaloïdes libres correspondants.

D'autres dérivés obtenus par modification chimique de la vinblastine ont été testés. Ainsi le sulfate de vinglycinate (I, sulfate, $R_1$=$OCH_3$, $R_2$=$COCH_2N(CH_3)_2$, Cancer Research 1967, *27*, 221—227) a été étudié en expérimentation clinique mais ne s'est généralement pas révélé supérieur à la vinblastine et à la vincristine.

Le brevet belge 813 168 décrit la vindésine (I, $R_1$=$NH_2$, $R_2$=H) et d'autres dérivés carboxamide en $C_3$ de la vinblastine. Des publications postérieures ont confirmé l'intérêt thérapeutique de la vindésine ou carboxamide-3 déacétyl-O-4 vinblastine, mais ce composé s'est cependant révélé inactif pour le traitement de tumeurs L 1210 transplantées chez la souris (C. J. Barnett et al., J. Med. Chem., *21*, 88, 1978).

Les dérivés de couplage d'acides aminés avec la vinblastine et d'autres alcaloïdes bisindoliques analogues ont été proposés d'une manière générale dans le brevet belge 813 168 susmentionné.

Néanmoins, aucun exemple de dérivé d'acide aminé spécifique n'a été divulgué et, conséquemment, aucune donnée physico-chimique n'a été fournie. Aucune méthode de préparation spécifique et aucun résultat pharmacologique particulier n'a été mentionné.

Il peut donc en être déduit que ces dérivés n'ont pas été réellement synthétisés et/ou n'ont pas été testés pour leur activité antitumorale.

Page 3, ligne 10 et suivantes, il est en outre mentionné que "l'activité anti-néoplastique semble être limitée à des structures très spécifiques et les chances d'obtenir des médicaments plus actifs en modifiant ces structures semblent également faibles."

Il a maintenant été constaté que les nouveaux composés décrits dans la présente invention sont capables de retarder efficacement la mort de souris auxquelles on a transplanté par voie intraveineuse des tumeurs P 388 et L 1210.

Alors que les alcaloïdes Vinca administrés par voie intraveineuse ne présentent généralement aucune activité sur la leucémie L 1210, les résultats obtenus montrent pour les nouveaux dérivés une activité très significative et inattendue.

Les activités sur les tumeurs expérimentales P 388 se sont également révélées, de manière surprenante, très supérieures à celles des alcaloïdes Vinca de référence. De nombreuse rémissions complètes ont été observées.

Les dérivés de l'invention possèdent en outre d'autres avantages importants et inattendus comparativement à la vinblastine et à ses analogues connus, en particulier la vindésine.

En particulier, les toxicités observées sont généralement inférieures à celles correspondant à la vindésine ou à la vincristine.

Les composés de l'invention répondent à la formule générale I;
dans laquelle

$R_1$ représente un ester d'acide aminé ou de peptide, couplé au fragment viblastinoyl-23 par un lien amide, le peptide comprenant de 1 à 6 acides aminés différents ou identiques, et le groupe ester ramifié ou non possédant de 2 à 9 atomes de carbone; et

$R_2$ représente un atome d'hydrogène ou un groupe acyle comportant de 2 à 9 atomes de carbone, ainsi que ses sels d'addition avec les acides minéraux ou organiques.

Les nouveaux composés de l'invention répondent plus particulièrement à la formule générale II

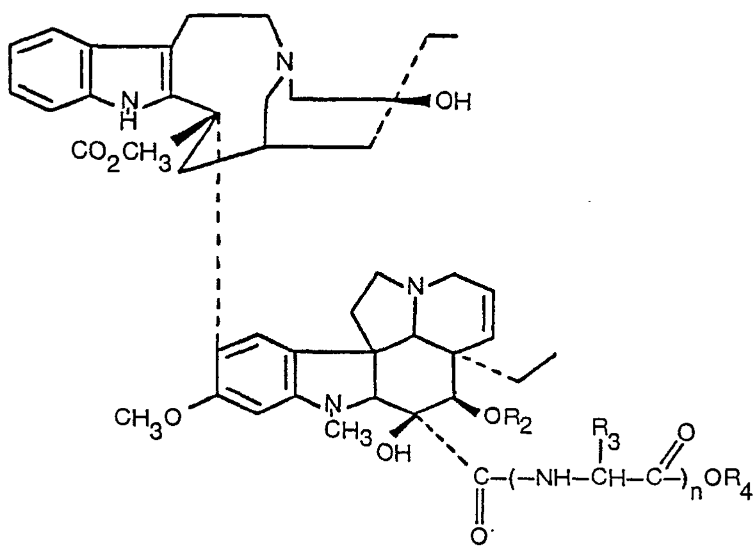

(II)

dans laquelle $R_2$ représente un atome d'hydrogène ou un groupe acyle comportant de 2 à 9 atomes de carbone; $R_3$ représente chaque fois indépendamment, un atome d'hyudrogène, un groupe alkyle ramifié ou non comportant de 1 à 8 atomes de carbone, un groupe hydroxy C1-C8 alkyle, amino-hydroxy C1-C8 alkyle, carboxy-C1-C8 alkyle, amido C1-C8 alkyle, guanidino C1-C8 alkyle, amino C1-C8 alkyle, thiol C1-C8 alkyle cystéinyl méthyle, thiométhyl-éthyl, benzyle, hydroxybenzyle

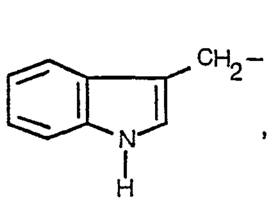 , 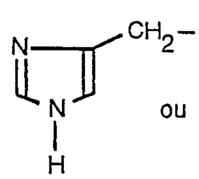 ou 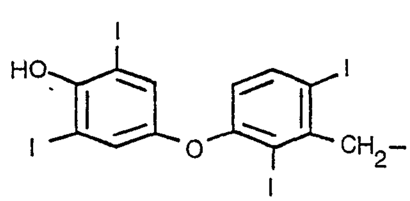

ou $R_3$ représente avec l'atome de carbone auquel il est attaché et l'azote du groupe amide, un cycle azole ou hydroxyazole;

n varie de 1 à 6;

et $R_4$ représente un groupe alkyle, linéaire ou ramifié possédant de 1 à 8 atomes de carbone, ou un groupe benzyle.

$COOR_4$ représente de préférence un groupe carboéthoxy ou carbométhoxy.

Plus particulièrement, le fragment structural de la formule II

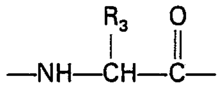

représente un radical de n'importe lequel des acides aminés naturels et de leurs isomères optiques de configuration D, notamment:

| | | | |
|---|---|---|---|
| la glycine | | acide aspartique | cystine |
| alanine | | acide glutamique | méthionine |
| valine | | asparagine | phénylalanine |
| leucine | | glutamine | tyrosine |
| isoleucine | | arginine | tryptophane |
| sérine | | lysine | proline |
| thréonine | | cystéine | histidine |
| hydroxyproline | ou | hydroxylysine. | |

On peut considérer ces composés comme étant des dérivés de la descarbométhoxy-3 désacétyl-O-4 vinblastine carboxamide-3. On préférera cependant les désigner comme étant des dérivés N-(vinblastinoyl-23) d'acide aminé.

Les composés préférés de l'invention répondent à la formule II dans laquelle $R_2$ représente un atome d'hydrogène et la partie acide aminé est dérivée d'un des acides aminés suivants: L ou D tryptophane, leucine, valine, isoleucine, alanine et phénylalanine. Parmi ces derniers, le L-tryptophane, la L-isoleucine et la L-alanine se sont révélés plus particulièrement intéressants.

Parmi les dérives d'esters peptidiques, les composés préférés répondent à la formule II dans laquelle n=2 et les acides aminés sont choisis dans la liste mentionnée ci-dessus, suivant n'importe quelle séquence.

En ce qui concerne les propriétés pharmacologiques, il est prévisible que de légères modifications de la structure du squelette peptide fourniront des composés d'activité comparable.

En particulier, la présence d'un acide $\alpha$-aminé non naturel (par exemple la norleucine, un acide $\alpha$-aminé N-monosubstitué ou un acide aminé, $\alpha,\alpha$-dialkyle) fournirait des composés d'activité similaire contre un certain nombre de tumeurs.

Pareils composés entrent donc dans le cadre de la présente invention.

Les nouveaux dérivés bisindoliques de l'invention sont obtenus à partir de la vinblastine par hydrazinolyse, nitrosation puis couplage avec l'ester d'acide aminé ou de polypeptide approprié.

Lorsque l'acide aminé ou le peptide contient des groupes fonctionnels dans la chaîne latérale $R_3$ (formule II), comme la lysine ou la cystéine, il peut être nécessaire de protéger ces groupes fonctionnels en utilisant les méthodes bien connues de l'homme de l'art.

Cette protection peut être effectuée, selon la nature du groupe à protéger, par le greffage d'un groupe benzyle, t-butyle, benzyloxycarbonyle, t-butoxycarbonyle, trifluoroacétyle ou trityle. D'autres agents bien connus en chimie peptidique peuvent être avantageusement utilisés.

Selon le procédé mentionné ci-dessus, les composés suivant l'invention peuvent être obtenus à partir de la vinblastine, de manière classique, par hydrazinolyse suivie de nitrosation, puis couplage avec l'ester d'acide aminé ou de peptide selon le schéma réactionnel I

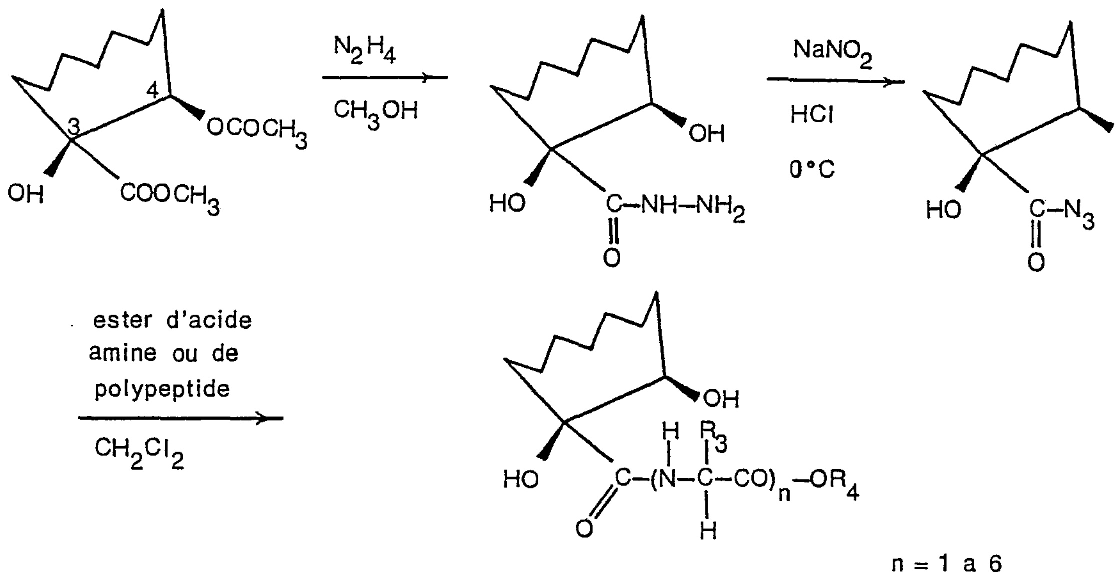


Schéma I

La première étape consiste à additionner un excès d'hydrazine anhydre à une solution de vinblastine base dans le méthanol anhydre. Le mélange réactionnel est alors chauffé sous atmosphère inerte pendant 12 à 30 heures à une température comprise entre 30 et 70°C. De préférence, la température sera maintenue vers 60°C, le temps de réaction étant alors de 24 heures.

L'hydrazide de la désacétyl-4-vinblastine est alors isolé par addition d'eau, extraction au dichlorométhane et concentration. Le composé peut être ultérieurement purifié par chromatographie préparative (silice neutre).

Lors de la deuxième étape, la fonction hydrazide de la vinblastine modifiée est transformée en azoture d'acide. Cette transformation est effectuée de manière connue, par addition de nitrite de sodium à l'hydrazide en solution dans un mélange eau-méthanol-acide.

L'acide utilisé peut être l'acide chlorhydrique.

La température de réaction est maintenue entre 0 et 5°C. Après extraction par un solvant aprotique non soluble dans l'eau, de préférence le chlorure de méthylène, la phase organique est partiellement concentrée.

L'azoture d'acide lui-même n'est de préférence pas isolé, mais directement mis en présence de l'ester d'acide aminé ou de polypeptide ou, le cas échéant, le dérivé correspondant protégé, en solution dans le chlorure de méthylène.

La quantité d'acide aminé mise en oeuvre est de 1 à 5 équivalents de vinblastine carboxazide.

Le milieu réactionnel est maintenu entre –3 et +10°C pendant 8 à 72 heures, le plus souvent environ 15 heures. La réaction est facilement suivie par chromatographie sur couche mince. Après concentration, on isole le composé de l'invention II qui peut être transformé en sulfate ou un autre sel d'acide minéral ou organique par cristallisation à partir d'une solution méthanolique de l'acide correspondant.

Les composés de l'invention sont purifiés en utilisant les techniques bien connues de chromatographie et de recristallisation.

Le cas échéant, le dérivé de désacétyl-0-4 vinblastine ainsi obtenu peut être réacétylé soit directement pour donner le dérivé de la vinblastine II dans lequel $R_2$=$COCH_3$ (J. Med. Chem. *22*, 391, 1979) ou par formation préliminaire du dérivé diacétoxy-3,4 suivie d'une hydrolyse sélective du groupe acétoxy en position 3. Le groupe hydroxyle en $C_4$ peut être, de manière connue en soi, estérifié par d'autres dérivés activés d'acides comportant de 2 à 9 atomes de carbone.

La présente invention s'étend également à la formulation pharmaceutique des composés bisindoliques nouveaux.

Les composés de l'invention présentent en particulier des propriétés antitumorales particulièrement intéressantes et susceptibles d'être utilisés en thérapeutique humaine.

Ces dérivés d'acide aminé peuvent être, en particulier, utilisés pour le traitement des leucémies du type L 1210, P 388, de gliomes, lymphosarcomes et d'autres leucémies ou tumeurs malignes.

En thérapeutique humaine, ils sont utilisés pour le traitement de la maladie de Hodgkin et pour d'autres tumeurs pouvant bénéficier d'un traitement avec la vinblastine, la vincristine et la vindésine.

Ces composés peuvent également être utilisés en médecine vétérinaire pour le traitement des tumeurs chez les animaux.

D'autres applications thérapeutiques intéressantes peuvent être envisagées pour ces nouveaux

dérivés d'alcaloïdes bisindoliques. Il a en effet été relevé que la vinblastine pourrait être utilisée dans le traitement de certaines arthrites (brevet des Etats-Unis d'Amérique n° 4 208 411) et la vincristine pour le traitement de psoriasis (brevet des Etats-Unis d'Amérique n° 3 749 784). L'activité anti-virale de certains alcaloides bis-indolique a également été relevée.

Pour le traitement des tumeurs malignes chez les animaux, les activités chimiothérapeutiques ont été testées en utilisant les sels de sulfate des composés de l'invention.

Des souris $DBA_2$ femelles de souche Charles River France ont été inoculées par voie intraveineuse avec $10^4$ cellules leucémiques provenant d'une ascite leucémique P 388 ou L 1210 de sept jours. Le jour 0 est le jour d'inoculation des cellules tumorales. Le composé de l'invention (sous forme sulfate) est alors injecté par voie intraveineuse en solution dans l'eau physiologique (NaCl 9/1000) suivant le schéma en une injection (jour 1) ou le schéma en trois injections (jours 1, 2 et 3). On calcule alors le MST (Median Survival Time), jour où la moitié des animaux sont morts.

Ce calcul est effectué le trentième jour.

Le pourcentage d'ILS (increased Life Span) ou pourcentage d'augmentation de survie est calculé à l'aide de la formule suivante:

$$\% \text{ ILS} = \left(\frac{\text{MST produit}}{\text{MST contrôles}} \times 100\right) - 100$$

Le nombre de souris survivant au trentième et au soixantième jour est également indiqué.

A des doses trop toxiques, le pourcentage d'ILS devient négatif, c'est-à-dire que les souris non traitées suivivent plus longtemps que celles ayant été injectées par la substance antitumorale.

Dans certains cas, les ILS observés dépendent de la variété de souris utilisée ($DBA_2$ France ou Etats-Unis d'Amérique).

Les résultats obtenus sont à comparer à ceux observés avec la vinblastine (VLB), la vindésine (VDS) et la vincristine (VCR) dans le tableau I ci-après. La supériorité pharmacologique des composés de l'invention est ainsi démontrée (tableaux II à XI ci-après).

Le tableau II fournit les résultats obtenus avec quelques dérivés de la leucine naturelle. Les dérivés suivants ont ainsi été testés:

VLE: N-(désacétyl-O-4 vinblastinoyl-23)-L-leucinate d'éthyle

VLM: N-(désacétyl-O-4 vinblastinoyl-23)-L-leucinate de méthyle.

Le tableau III rassemble les résultats obtenus avec un dérivé de la D-leucine, en l'occurrence le N-(désacétyl-O-4 vinblastinoyl-23) D-leucinate d'éthyle (VDLE).

Les résultats obtenus avec les dérivés suivants du L-tryptophane sont repris dans le tableau IV:

V-Trypt E: N-(désacétyl-O-4 vinblastinoyl-23)-L-tryptophanate d'éthyle, MST calculé à 30 et à 60 jours;

V-Trypt M: N-(désacétyl-O-4 vinblastinoyl-23)-L-tryptophanate de méthyle.

Le tableau V présente les activités observées avec le dérivé du tryptophanate d'éthyle de configuration absolue D (VD-Trypt E).

Les tableaux VI à XI reprennent les résultats obtenus avec les composés suivants:

VAE: N-(désacétyl-O-4 vinblastinoyl-23)-L-alaninate d'éthyle (tableau VI)

VPE: N-(désacétyl-O-4 vinblastinoyl)-L-phénylalaninate d'éthyle (tableau VII)

VILE: N-(désacétyl-O-4 vinblastinoyl-23)-L-isoleucinate d'éthyle (tableau VIII)

VILM: N-(désacétyl-O-4 vinblastinoyl-23)-L-isoleucinate de méthyle (tableau VIII)

VVE: N-(désacétyl-O-4 vinblastinoyl-23)-L-valinate d'éthyle (tableau IX)

V-Tyr E: N-(désacetyl-O-4 vinblastinoyl-23)-L-tyrosinate d'éthyle (tableau X)

V-Val-Trypt E: N-(désacétyl-O-4 vinblastinoyl-23)-L-valinyl-L-tryptophanate d'éthyle (tableau XI).

TABLEAU I

(Vinblastine (VLB)
P388

| Dose mg/kg/j | Schéma | Nombre d'animaux | MST max 30 j | ILS % | > 30 j | > 60 j |
|---|---|---|---|---|---|---|
| 4 | 1 | 10 | 14,6 | 36,4 | 0 | 0 |
| 6 | 1 | 10 | 15,6 | 45,8 | 0 | 0 |
| 8 | 1 | 9 | 18,5 | 72,9 | 0 | 0 |

TABLEAU I (suite)

Vindesine (VDS)
P 388

| Dose mg/kg/j | Schéma | Nombre d'animaux | MST max 30 j | ILS % | > 30 j | > 60 j |
|---|---|---|---|---|---|---|
| 2 | 1 | 10 | 13,6 | 27,1 | 0 | 0 |
| 3 | 1 | 10 | 14 | 30,8 | 0 | 0 |
| 4 | 1 | 10 | 14,8 | 38,3 | 0 | 0 |
| 5 | 1 | 10 | 13,8 | 30,2 | 0 | 0 |
| 6 | 1 | 10 | 14 | 32 | 0 | 0 |

L 1210

| | | | | | | |
|---|---|---|---|---|---|---|
| 3 | 1 | 20 | 8,7 | 5 | 0 | 0 |

Vincristine (VCR)
P 388

| Dose mg/kg/j | Schéma | Nombre d'animaux | MST max 30 j | ILS % | > 30 j | > 60 j |
|---|---|---|---|---|---|---|
| 0,5 | 1 | 10 | 11,56 | 4 | 0 | 0 |
| 1 | 1 | 19 | 12,32 | 15 | 0 | 0 |
| 1,5 | 1 | 20 | 12,78 | 19 | 0 | 0 |
| 2 | 1 | 10 | 6 | –46 | 0 | 0 |

L 1210

| | | | | | | |
|---|---|---|---|---|---|---|
| 0,5 | 1 | 10 | 7,56 | 1 | 0 | 0 |
| 1 | 1 | 20 | 7,91 | 5 | 0 | 0 |
| 1,5 | 1 | 20 | 8,38 | 12 | 0 | 0 |
| 2 | 1 | 10 | 8,67 | 16 | 0 | 0 |

TABLEAU II
DERIVES DE LA LEUCINE NATURELLE

VLE
P 388

| Dose mg/kg/j | Schéma | Nombre d'animaux | MST max 30 j | ILS % | > 30 j | > 60 j |
|---|---|---|---|---|---|---|
| 20 | 1 | 10 | 16 | 52,4 | 2 | 1 |
| 22 | 1 | 10 | 20 | 90,5 | 4 | 1 |
| 24 | 1 | 20 | 18 | 65 | 2 | 0 |
| 26 | 1 | 10 | 17,5 | 53,5 | 1 | 0 |
| 28 | 1 | 10 | 19,6 | 71,9 | 1 | 1 |
| 30 | 1 | 10 | 21 | 84,2 | 2 | 1 |
| 34 | 1 | 10 | 6 | –48 | 2 | 1 |
| 36 | 1 | 10 | 5,4 | –53 | 1 | 1 |
| 5 | 1,2,3 | 9 | 14,5 | 25 | 0 | 0 |
| 6 | 1,2,3 | 10 | 15 | 29,3 | 0 | 0 |
| 7 | 1,2,3 | 10 | 14,2 | 24,6 | 0 | 0 |
| 9 | 1,2,3 | 10 | 5 | –56 | 1 | 1 |
| 12 | 1,2,3 | 9 | 4,8 | –59 | 0 | 0 |

VLM
P 388

| Dose mg/kg/j | Schéma | Nombre d'animaux | MST max 30 j | ILS % | > 30 j | > 60 j |
|---|---|---|---|---|---|---|
| 10 | 1 | 20 | 16,7 | 41 | 2 | 1 |
| 10,5 | 1 | 10 | 16,4 | 53,3 | 0 | 0 |
| 11 | 1 | 19 | 17,7 | 51 | 2 | 1 |
| 11,5 | 1 | 10 | 16,8 | 57 | 0 | 0 |
| 12 | 1 | 20 | 17,5 | 49 | 3 | 2 |
| 12,5 | 1 | 30 | 17,3 | 49 | 8 | 3 |
| 13 | 1 | 20 | 20,5 | 74 | 7 | 5 |
| 15 | 1 | 10 | 18 | 55,2 | 4 | 1 |

L 1210

| | | | | | | |
|---|---|---|---|---|---|---|
| 10,5 | 1 | 10 | 10,6 | 24,7 | 0 | 0 |
| 11,5 | 1 | 10 | 10,8 | 27 | 0 | 0 |
| 12,5 | 1 | 10 | 10 | 17,6 | 0 | 0 |

TABLEAU III

VDLE
P 388

| Dose mg/kg/j | Schéma | Nombre d'animaux | MST max 30 j | ILS % | > 30 j | > 60 j |
|---|---|---|---|---|---|---|
| 6 | 1 | 10 | 14,7 | 26,7 | 0 | 0 |
| 8 | 1 | 10 | 17 | 46,5 | 2 | 1 |
| 10 | 1 | 20 | 28,5 | 146 | 9 | 2 |
| 11 | 1 | 10 | 16 | 49,5 | 1 | 0 |
| 12,5 | 1 | 10 | 30 | 145,9 | 6 | 3 |

L 1210

| | | | | | | |
|---|---|---|---|---|---|---|
| 9 | 1 | 10 | 10,8 | 27 | 0 | 0 |
| 10 | 1 | 10 | 11 | 29,4 | 0 | 0 |

TABLEAU IV

V-Trypt-E
P 388

| Dose mg/kg/j | Schéma | Nombre d'animaux | MST max 30 j | ILS % | > 30 j | > 60 j |
|---|---|---|---|---|---|---|
| 20 | 1 | 10 | 11,4 | 43,9 | 0 | 0 |
| 40 | 1 | 10 | 17 | 58,8 | 5 | 4 |
| 50 | 1 | 20 | 24,5 | 117 | 9 | 5 |
| 55 | 1 | 60 | 41 | 279 | 39 | 25 |
| 60 | 1 | 100 | 33 | 202 | 57 | 36 |
| 65 | 1 | 29 | 22,75 | 114 | 13 | 9 |
| 70 | 1 | 20 | 6,5 | –39 | 8 | 5 |

L 1210

| | | | | | | |
|---|---|---|---|---|---|---|
| 55 | 1 | 30 | 11,8 | 56 | 0 | 0 |
| 60 | 1 | 30 | 12,5 | 65 | 2 | 2 |
| 65 | 1 | 30 | 12 | 62 | 0 | 0 |
| 70 | 1 | 10 | 12,3 | 61,8 | 1 | 1 |

TABLEAU IV (suite)

P 388 (ILS calculé au jour 60)

| Dose mg/kg/j | Schéma | Nombre d'animaux | MST max 30 j | ILS % | > 30 j | > 60 j |
|---|---|---|---|---|---|---|
| 55 | 1 | 30 | 60 | 457 | 28 | 16 |
| 60 | 1 | 60 | 36 | 224 | 52 | 24 |

V-Trypt-M
P 388

| Dose mg/kg/j | Schéma | Nombre d'animaux | MST max 30 j | ILS % | > 30 j | > 60 j |
|---|---|---|---|---|---|---|
| 30 | 1 | 10 | 15 | 33,9 | 0 | 0 |
| 40 | 1 | 10 | 16 | 42,8 | 0 | 0 |
| 50 | 1 | 10 | 23,5 | 199,8 | 1 | 0 |
| 60 | 1 | 20 | 26 | 140 | 7 | 2 |
| 70 | 1 | 10 | 26 | 145,3 | 5 | |

L 1210

| | | | | | | |
|---|---|---|---|---|---|---|
| 50 | 1 | 10 | 11,5 | 47.4 | 0 | 0 |
| 55 | 1 | 10 | 12.5 | 60.2 | 0 | 0 |

TABLEAU V

VD-Trypt-E
P 388

| Dose mg/kg/j | Schéma | Nombre d'animaux | MST max 30 j | ILS % | > 30 j | > 60 j |
|---|---|---|---|---|---|---|
| 20 | 1 | 10 | 13,3 | 18,7 | 0 | |
| 30 | 1 | 10 | 15,8 | 41,4 | 1 | |
| 40 | 1 | 20 | 16,5 | 52 | 1 | |
| 50 | 1 | 10 | 22 | 107,5 | 1 | |
| 60 | 1 | 10 | 4,4 | –60,7 | 1 | |

L 1210

| | | | | | | |
|---|---|---|---|---|---|---|
| 40 | 1 | 10 | 9,5 | 21,8 | 0 | 0 |
| 45 | 1 | 10 | 10,5 | 34,6 | 0 | 0 |

TABLEAU VI

VAE
P 388

| Dose mg/kg/j | Schéma | Nombre d'animaux | MST max 30 j | ILS % | > 30 j | > 60 j |
|---|---|---|---|---|---|---|
| 5 | 1 | 10 | 14,8 | 27,6 | 0 | 0 |
| 10 | 1 | 10 | 16,3 | 40,5 | 0 | 0 |
| 12,5 | 1 | 19 | 16,2 | 44 | 1 | 1 |
| 15 | 1 | 20 | 17 | 46 | 5 | 4 |
| 25 | 1 | 10 | 19 | 63,8 | 2 | 1 |

L 1210

| | | | | | | |
|---|---|---|---|---|---|---|
| 12,5 | 1 | 20 | 10,4 | 48 | 0 | 0 |

TABLEAU VII

VPE
P 388

| Dose mg/kg/j | Schéma | Nombre d'animaux | MST max 30 j | ILS % | 30 j | 60 j |
|---|---|---|---|---|---|---|
| 10 | 1 | 10 | 13 | 12,1 | 0 | 0 |
| 20 | 1 | 10 | 14,4 | 24,1 | 0 | 0 |
| 40 | 1 | 10 | 30 | 158,6 | 7 | 7 |
| 50 | 1 | 30 | 13,6 | 26 | 10 | 7 |
| 55 | 1 | 10 | 14,6 | 36,4 | 1 | 0 |

L 1210

| | | | | | | |
|---|---|---|---|---|---|---|
| 55 | 1 | 10 | 8,5 | 0 | 0 | 0 |
| 60 | 1 | 10 | 9 | 13,9 | 0 | 0 |
| 65 | 1 | 10 | 8,8 | 3,5 | 0 | 0 |
| 70 | 1 | 20 | 8,7 | 13 | 0 | 0 |
| 75 | 1 | 10 | 9,5 | 25 | 0 | 0 |

TABLEAU VIII

VILE
P 388

| Dose mg/kg/j | Schéma | Nombre d'animaux | MST max 30 j | ILS % | >30 j | >60 j |
|---|---|---|---|---|---|---|
| 8 | 1 | 30 | 30 | 172 | 20 | 2 |
| 9 | 1 | 20 | 30 | 179 | 12 | |

L 1210

| | | | | | | |
|---|---|---|---|---|---|---|
| 8 | 1 | 20 | 11,57 | 58 | 0 | 0 |
| 9 | 1 | 10 | 12,8 | 68,4 | 0 | 0 |

VILM
P 388

| Dose mg/kg/j | Schéma | Nombre d'animaux | MST max 30 j | ILS % | >30 j | >60 j |
|---|---|---|---|---|---|---|
| 3 | 1 | 10 | 16,4 | 41,3 | 0 | 0 |
| 5 | 1 | 29 | 18,2 | 68 | 8 | 3 |
| 6 | 1 | 69 | 23,4 | 115 | 25 | 6 |
| 7 | 1 | 30 | 30 | 117 | 16 | 5 |
| 9 | 1 | 10 | 6 | –48,3 | 4 | 4 |

L 1210

| | | | | | | |
|---|---|---|---|---|---|---|
| 6 | 1 | 29 | 11,2 | 60 | 0 | 0 |

TABLEAU IX

VVE
P 388

| Dose mg/kg/j | Schéma | Nombre d'animaux | MST max 30 j | ILS % | > 30 j | > 60 j |
|---|---|---|---|---|---|---|
| 5 | 1 | 10 | 13,6 | 17,2 | 0 | 0 |
| 10 | 1 | 10 | 17,8 | 53,4 | 0 | 0 |
| 12,5 | 1 | 10 | 21 | 82,6 | 2 | 0 |
| 14 | 1 | 20 | 21 | 98 | 3 | |
| 15 | 1 | 39 | 23,5 | 107 | 14 | 4 |
| 17,5 | 1 | 9 | 8,4 | –22,2 | 0 | 0 |
| 25 | 1 | 10 | 6,5 | –44 | 0 | 0 |

L 1210

| | | | | | | |
|---|---|---|---|---|---|---|
| 15 | 1 | 19 | 11,4 | 61,9 | 0 | 0 |

TABLEAU X

V-Tyr E
P 388

| Dose mg/kg/j | Schéma | Nombre d'animaux | MST max 30 j | ILS % | > 30 j | > 60 j |
|---|---|---|---|---|---|---|
| 20 | 1 | 10 | 13,9 | 29,9 | 0 | 0 |
| 40 | 1 | 10 | 16,4 | 53,3 | 1 | 1 |
| 50 | 1 | 10 | 16 | 49,5 | 1 | 1 |
| 60 | 1 | 10 | 16 | 49,5 | 3 | 2 |
| 70 | 1 | 10 | 5,4 | –49,5 | 0 | 0 |

TABLEAU XI

V-Val-Trypt E
P 388

| Dose mg/kg/j | Schéma | Nombre d'animaux | MST max 30 j | ILS % | > 30 j | > 60 j |
|---|---|---|---|---|---|---|
| 15 | 1 | 10 | 26 | 132,1 | 3 | 0 |
| 25 | 1 | 10 | 27,5 | 145,5 | 3 | 2 |
| 35 | 1 | 19 | 27,5 | 154 | 8 | 4 |
| 45 | 1 | 10 | 30 | 183 | 8 | 4 |
| 50 | 1 | 9 | 27,5 | 145,5 | 4 | 3 |

TABLEAU XII

P 388 injectée par la voie intrapéritonéale
BDFI femelles Charles River, France

| Produit | Dose mg/kg/j | Schéma | Nombre d'animaux | MST* | ILS* % | Pourcentage de survivants au jour 30 / nombre total d'animaux |
|---|---|---|---|---|---|---|
| VCR | 2,7 | 1 | 11 | 15,6 | 64,2 | 0 |
| V-Trp-E | 50 | 1 | 10 | 22 | 131,6 | 1 |
| V-Trp-E | 60 | 1 | 10 | 31 | 226 | 6 |
| V-Trp-E | 80 | 1 | 10 | 21,5 | 126,3 | 0 |

Activité du V-Trp-E et de VCR sur la leucémie P 388 implantée en i.p. chez des souris BDF1 femelles. Des souris BDF1 femelles reçoivent $10^6$ cellules leucémiques au jour 0. Au jour 1, les produits sont administrés par voie i.p. aux doses précisées.

MST* = Median Survival Time = jour moyen de survie
% ILS* = Increase in Life Span = % d'augmentation de la durée de survie.

*"Lewis Lung Carcinoma" (3LL)*

1,1 x $10^6$ cellules tumorales sont injectées intramusculairement dans la patte postérieure droite de souris C57B1 femelles.

Les drogues sont administrées par voie intraveineuse suivant un schéma en 3 injections.

Les animaux sont sacrifiés le 23e jour après la greffe. Le poids de la tumeur primaire est estimé en grammes; les métastases pulmonaires sont comptées et leur diamètre mesuré. Le poids des métastases est évalué en supposant qu'il s'agit de sphères de densité égale à 1; le nombre de souris sans métastase est également relevé.

Le tableau XIII reprend les résultats obtenus. Les produits présentent une activité sur la tumeur primaire.

Le poids moyen des métastases, leur nombre moyen ainsi que le nombre de souris sans métastase sont indiqués et permettent de juger l'activité anti-métastasique des composés anti-tumoraux administrés.

TABLEAU XIII

| Produit | Dose mg/kg/j | Nombre de souris disséquées | Poids de la tumeur (g) $\bar{X}$ | Poids de la tumeur (g) $\sigma$ | Poids des métastases (mg) $\bar{X}$ | Poids des métastases (mg) $\sigma$ | Nombre moyen de métastases $\bar{X}$ | Nombre moyen de métastases $\sigma$ | Nombre de souris sans métastase |
|---|---|---|---|---|---|---|---|---|---|
| — | — | 20 | 9,64 | 0,916 | 27,61 | 54,53 | 28,4 | 15,22 | 0 |
| VDS | 2,5 | 22 | 5,72 | 0,698 | 0,40 | 0,91 | 13,68 | 7,49 | 0 |
| VTrypE | 50 | 21 | 4,83 | 0,868 | 0,047 | 0,038 | 4,58 | 2,83 | 2 |

Des souris C57B1 femelles reçoivent, au jour, 0 1, 1 x $10^6$ cellules tumorales par voie intramusculaire. Aux jours 1, 8 et 15, les drogues sont administrées aux doses indiquées. Le 23e jour les souris sont sacrifiées; la tumeur primaire est pesée, les métastases pulmonaires sont numérotées et leur diamètre est mesuré.

Les activités antitumorales rassemblées dans les tableaux I à XII confirment l'efficacité inattendue des dérivés d'acides aminés de la présente invention. La plupart des dérivés se révèlent effectivement être supérieurs à la vindésine, tant pour les tumeurs injectées par voie i.v. que par voie i.p. (tableau XII). L'activité exceptionnelle de ces dérivés sur les tumeurs L 1210 est démontrée.

Il y a lieu de noter que, parmi les tumeurs expérimentales actuellement accessibles, la leucémie L 1210 est reconnue comme étant une des tumeurs expérimentales les plus significatives pour la sélection de médicaments antitumoraux chez l'homme.

Le tableau XIII reprend les résultats obtenus à l'occasion d'un test antimétastasique. L'activité du dérivé VLB-Trypt-E se révèle très supérieure à celle de la vindésine. L'efficacité sur la tumeur primaire est en outre comparable à celle de la vindésine.

On notera en particulier l'activité exceptionnelle du sulfate de N-(désacétyl-O-4 vinblastinoyl-23) tryptophanate d'éthyle qui a fourni un ILS calculé à 60 jours (P 388) de 457% avec la moitié des souris survivant plus longtemps. La dose optimale se situerait alors aux environs de 60 mg. Par contre, les dérivés d'acides aminés qui ont été testés sous la forme amide ou acide ne présentent que des activités faibles ou non significatives. Ces dérivés peuvent cependant être utilisés comme intermédiaires utiles pour l'obtention d'autres alcaloïdes bisindoliques très actifs.

En général, les composés de l'invention se sont révélés être beaucoup moins toxiques que les alcaloïdes Vinca actuellement utilisés en thérapeutique anticancéreuse. La dose létale 50 ($DL_{50}$) de la VLE a été déterminée en utilisant des souris femelles de souche Charles River $CD_1$ et pesant au moins 24 g. La méthode de calcul de Litchfield et Willcoxon fournit une valeur de $DL_{50}$ de 32 mg/kg.

Les doses correspondantes pour la vinblastine et la vindésine sont respectivement de 24 mg/kg et environ 11 mg/kg. On a noté en particulier contrairement aux cas de la vinblastine et de la désacétyl vinblastine, l'absence de toxicité hépatique à des doses de 20 à 40 mg/kg.

Les toxicité aiguës du V-Trp-E et du VILE ont été également déterminées sur des souris NMRI femelles. Les valeurs suivantes ont été obtenues : V-Trp-E : 100,8 mg/kg, VILE : 17,7 mg/kg à comparer avec les valeurs correspondantes de la vinblastine et de la vindésine, c'est-à-dire respectivement 27,4 et 13,8 mg/kg. Le V-Trp-E est donc nettement moins toxique que la VLB ou la VDS.

Pour leur application thérapeutique, les composés de l'invention, éventuellement sous forme lyophilisée, peuvent être utilisés par la voie orale ou par administration parenterale par exemple par voie intra-veineuse, dissous dans un diluant pharmaceutiquement acceptable, sous la forme d'une base ou d'un sel d'addition d'un acide pharmaceutiquement acceptable. Parmi ces sels d'addition, on préférera les sels non toxiques tels que les sels d'acides minéraux comme l'acide chlorhydrique, l'acide phosphorique et l'acide sulfurique, ou d'acides organiques comme l'acide acétique, propionique, succinique, tartrique, oxalique, méthanesulfonique, benzènesulfonique.

L'eau physiologique ou d'autres solutions salines tamponnées, par exemple avec un phosphate, sont des solvants appropriés. D'une manière générale, ces composés peuvent être utilisés en s'inspirant des techniques et des limitations qui sont connues pour les traitements thérapeutiques avec les autres alcaloïdes de la classe des Vinca.

La substance active est généralement administrée à une posologie pouvant varier de 0,01 mg à 5 mg/kg, en fonction du dérivé utilisé et du schéma thérapeutique adopté. Les doses hebdomadaires totales varieront généralement de 0,1 mg à 35 mg/kg.

Les compositions selon l'invention sont présentées sous des formes d'administration contenant le principe actif à la dose unitaire de 2 à 900 mg.

Les composés de l'invention peuvent être utilisés seuls ou en combinaison avec un ou plusieurs agents carcinostatiques incluant par exemple les agents alkylants, les antimétabolites tels que le méthotrexate, le fluoro-5-uracile, la mercapto-6 purine, la thio-6 guanine, les arabinosides de la cytosine et les antibiotiques tels que l'actinomycine D, la daunorubicine, l'adriamycine, et le cis-diamino-dichloro-platine. En particulier, ces nouveaux dérivés de la vinblastine peuvent être utilisés en association entre eux.

Les exemples suivants illustrent le procédé conduisant aux composés de l'invention.

*Mode opératoire général pour l'obtention de dérivés N-(désacétyl-O-4-vinblastinoyl-23) d'acides aminés.*

1 g (1.3 $10^{-3}$ M) de descarbométhoxy-3-désacétyl-O-4 vinblastine carboxhydrazide-3 est solubilisé dans 23 ml de méthanol absolu et 74 ml d'acide chlorhydrique 1N. Cette solution est alors refroidie à $-10$°C et 207 mg de nitrite de sodium y sont ajoutés en une fois.

Le mélange réactionnel est abandonné alors pendant 10 à 30 minutes à 0°C. Après un contrôle par chromatographie sur couche mince, le milieu réactionnel est alcalinisé à pH 8,5 à $-10$°C, par une solution saturée de bicarbonate de sodium.

La solution alcaline est extraite à 0°C avec son propre volume en chlorure de méthylène refroidi à 0°C jusqu'à obtention d'une réaction de Meyer négative sur la phase aqueuse. Les phases organiques sont rassemblées, séchées sur $Na_2SO_4$ et filtrées à 0°C.

On y ajoute alors $1.43.10^{-3}$ M (1,1 équivalent) de l'ester aminé retenu et on concentre sous vide jusqu'à un volume de 4 ml environ. Cette solution est alors conservée pendant 24 à 48 h à 4°C. L'évolution de la réaction est suivie par la chromatographie sur couche mince.

La solution est alors tirée à sec et fournit 1,05 g de produit de couplage brut qui est quasi unitache à la chromatographie sur couche mince.

*Purification.*

Le résidu sec obtenu ci-dessus est placé sur une colonne de gel de silice et élué avec un mélange éther-méthanol saturé en ammoniac 96% + 4%.

On recueille des fractions de 10 ml et on les teste en chromatographie sur couche mince. La révélation se fera à la ninhydrine (acides aminés) et au cérique (alcaloïdes).

Lorsque les acides aminés sont passés (ninhydrine-), on élue encore avec 100 $cm^3$ du solvant employé avant de la changer par un mélange 92% — 8%.

L'alcaloïde couplé à l'acide aminé est alors récolté (cérique +).

Les fractions identiques sont rassemblées, concentrées à sec au Rotavapor, reprises au chlorure de méthylène, séchées sur sulfate de sodium, filtrées et évaporées à sec. La mousse obtenue est l'alcaloïde dimère couplé avec l'acide aminé tout pur dont les données physico-chimiques seront faites sur une partie aliquote. Le restant sera directement transformé en sulfate. Les rendements en base des différents dérivés sont donnés à titre indicatif et susceptibles d'amélioration.

*Préparation du sulfate*

La base amorphe (mousse) obtenue ci-dessus est reprise dans 20 fois son poids en éthanol.

A cette solution, on ajoute alors, très lentement et sous vive agitation, 2 équivalents d'acide sulfurique provenant d'une solution 2% $H_2SO_4$/98% éthanol anhydre. (0.484 équivalent/litre). Après l'addition des 2 équivalents, on laisse encore agiter pendant une demi-heure avant de concentrer sous pression réduite. Par addition d'éther sulfurique anhydre, sous vive agitation, le sulfate du dérivé engagé précipite. Filtration et séchage sous vide à 10°C fournissent le sulfate du dérivé couplé prêt à l'emploi.

La numérotation adoptée pour la description des spectres de résonance magnétique nucléaire des exemples qui suivent est inspirée de celle proposée par Le Men et Taylor pour les dérivés du type aspidospermidine (Experientia 21, 508, 1965).

## Exemple 1

*Préparation de la vinblastine (VLB) base*

A une solution de 1,5 g de sulfate de VLB ($1.65.10^{-3}$M) dissous dans 15 cc d'eau distillée, sont

ajoutés successivement sous vive agitation, 15 cc de chlorure de méthylène ainsi que 1,5 cc d'ammoniaque concentrée. Après cinq minutes, le mélange est décanté et la phase aqueuse extraite avec encore 3 x 15 cc de chlorure de méthylène. Les phases organiques rassemblées sont lavées avec 2 x 40 cc d'eau désionisée, séchées sur sulfate de sodium anhydre puis évaporées à sec au rotavapor. On recueille ainsi 1,32 g de VLB base (99%).

*Préparation de la descarbométhoxy-3-désacétyl-O-4 vinblastine carboxhydrazide-3 (VLH)*

A une solution de 1 g de VLB base (1.23 $10^{-3}$M) dans 7 ml d'éthanol anhydre, on ajoute 14 ml d'hydrazine anhydre et 7 ml d'éthanol anhydre. Le mélange réactionnel ainsi formé est maintenu vingt heures à 60°C.

Après refroidissement, on ajoute 28 ml d'eau saturée en sel et on extrait avec un même volume de chlorure de méthylène jusqu'à obtention d'une réaction de Meyer négative sur la phase aqueuse acidifiée.

Les phases organiques sont séchées sur $MgSO_4$ et évaporées à sec sous pression réduite.

L'hydrazide obtenu avec 88% de rendement (0,760 g) est unitache en chromatographie sur couche mince.

Exemple 2

*Synthèse de la N-(désacétyl-O-4 vinblastinoyl-23)-L-leucinate d'éthyle (VLE)*

A une solution de 275 mg (3,58 $10^{-4}$M) de O—4 désacétyl vinblastine hydrazide dans 7 ml de méthanol anhydre et 20,5 ml d'HCl 1N, refroidie préalablement à 0°C, on ajoute 58 mg de nitrite de sodium.

On laisse agiter pendant 25 minutes avant de revenir à un pH de 8,5 par addition d'une solution de bicarbonate de sodium à 5%. L'azoture formé est extrait au chlorure de méthylène.

La phase organique est séchée sur $MgSO_4$ et filtrée. On y ajoute alors 68,4 mg de leucinate d'éthyle (4,29.$10^{-4}$ M) et on concentre sous vide, jusqu'à l'obtention de 4 ml environ.

Cette solution est alors laissée pendant 24 heures au réfrigérateur.

La réaction étant alors terminée, on ajoute 50 ml de chlorure de méthylène et on lave plusieurs fois avec le même volume d'eau déminéralisée puis une fois avec une solution saturée en NaCl.

Les phases organiques sont séchées sur $MgSO_4$, filtrées puis évaporées sous vide.

On recueille ainsi 300 mg de produit brut auxquels on ajoute 0,035 g de $H_2SO_4$ en solution dans 1 ml de méthanol anhydre.

Le sel formé est précipité à l'éther.

Le précipité est lavé dix fois avec 50 cc d'éther diéthylique anhydre.

Le résidu, 183 mg (57%) ne contient que du sulfate de VLE pratiquement pur et exempt de leucinate d'éthyle.

Après libération des bases, ces dernières peuvent être chromatographiées sur gel de silice (10 g de $SiO_2$) et éluées avec 50 cc d'éther/MeOH-$NH_3$ sat. (92%/8%) puis 250 ml d'éther/MeOH-$NH_3$ (85%/15%). La VLE est obtenue pure en tête du second éluat. On recueille ainsi 49% de VLE purifiée soit 152 mg de base.

Propriétés physico-chimiques de la VLE

Point de fusion: ~ 169°C

$[\alpha]D^{CHCl_3}_{c\,=\,0,35}$: 60°

Spectre ultraviolet (MeOH, $\lambda$ max, nm, log $\varepsilon$):

221 (4,62); 267 (4,15); 287 (4,02); 295 (3,99).

Spectre infra-rouge (KBr, $cm^{-1}$):

3470, 2960, 2880, 1735, 1665, 1610.

Spectre de masse (m/e, %):

924 (6) $M^+$ +28; 910 (56) $M^+$ +14; 897 (62); 896 (100); 865 (25); 938 (68); 772 (19); 709 (25); 651 (43); 571 (69).

Spectre de résonance magnétique nucléaire ($H^1$, $CDCl_3$, ppm, 360MHz):

9,66 (1H, bs, $C^{16}$-OH); 8,20 (1H,s,$N'^{\alpha}$H); 7,52 (1H,d); 7,15 (3H,m); 6,56 (1H,s,$C^9$-H); 6,05 (1H,s,$C^{12}$-H); 5,86 (1H,dd,$C^{14}$-H, J 14-15 = 12; J14-3 = 3,6; 5,78 (1H,d,$C^{15}$-H); 4,69 (1H,m,CH(NHR)CO—); 4,2 (2H,q,*$OCH_2$* $CH_3$); 4,18 (1H,t,$C^{17}$-H); 3,77 (3H,s,$OCH_3$); 3,66 (3H,s,$OCH_3$); 3,47 (1H,s,$C_5$-H); 2,77 (3H,s,$N^{\alpha}$-$CH_3$); 0,92 (12H,m,-$C^{18}H_3$ + $C^{18'}H_3$ + isopropyl).

Exemple 3

N-(désacétyl-O-4 vinblastinoyl-23)-L-leucinate de méthyle (VLM)

En appliquant le mode opératoire général on obient la VLM avec un rendement de 68%.

Propriétés physico-chimiques de la VLM

Point de fusion: ~ 172°C

$[\alpha]D^{CHCl_3}_{c=0,27}$: 67°

Spectre ultraviolet ($CH_3OH$, $\lambda$ max, nm, log $\varepsilon$):

220 (4,61); 267 (4,15); 287 (4,03); 294 (3,98).

Spectre infra-rouge (KBr, $cm^{-1}$):

3475; 2960; 2880; 1740; 1680; 1615.

Spectre de masse (m/e, %):

910 (25) $M^+$+28; 896 (78) $M^+$+14; 883 (26) $M^+$+1; 882 (36) $M^+$; 850 (29); 836 (41); 822 (100); 708 (15); 681 (56); 650 (78); 570 (70).

Spectre de résonance magnétique nucléaire ($CDCl_3$, ppm, 60 MHz);

9,21 (1H,s,$C^{16}$-OH); 8,1 (1H,s,$N'^{\alpha}$-H); 7,53 (1H,m); 7,23 (3H,m); 6,63 (1H,s,$C^9$-H); 6,13 (1H,s,$C^{12}$-H); 5,86 (2H,m,$C^{14}$-H + $C^{15}$-H); 3,83 (3H,s, —$OCH_3$); 3,80 (3H,s, —$COOCH_3$); 3,63 (3H,s,—$OCH_3$); 2,8 (3H,s,-$N^{\alpha}$-$CH_3$); 0,96 (12H,m, $C^{18}H_3$ + $C^{18'}H_3$ + isopropyl).

Exemple 4

N-(désacetyl-O-4 vinblastinoyl-23)-L-leucinate de n-butyle (VLn-But)

En appliquant le mode opératoire général on obtient la VLn-But avec un rendement de 58%.

Propriétés physico-chimiques de la VLn-But

Point de fusion: ~ 158°C

$[\alpha]D^{CHCl_3}_{c=0,26}$: 74°

Spectre ultraviolet ($CH_3OH$, $\lambda$ max, nm, log $\varepsilon$):

220 (4,66); 266 (4,21); 289 (4,08); 296 (4,03).

Spectre infra-rouge (KBr, $cm^{-1}$):

3470, 2960, 2880, 1740, 1670, 1615.

Spectre de masse (m/e, %):

952 (5) $M^+$ + 28; 938 (31) $M^+$ + 14; 924 (12) $M^+$; 923 (15) $M^+$-1; 891 (13); 863 (36); 835 (5); 821 (11); 708 (33); 650 (81); 570 (100).

Spectre de résonance magnétique nucléaire ($CDCl_3$,ppm,60MHz):

9,6 (1H,s,$C^{16}$-OH); 8,06 (1H,s,$N'^{\alpha}$-H); 7,5 (1H,m); 7,2 (3H,m); 6,63 (1H,s,$C^9$-H); 6,1 (1H,s,$C^{12}$-H); 5,86 (2H,m,$C^{14}$-H + $C^{15}$-H); 4,2 (2H,t,—COO—$CH_2$); 3,83 (3H,s,—$OCH_3$); 3,63 (3H,s,—$OCH_3$); 2,8 (3H,s,—N—$CH_3$); 1 (15H,m,—$C^{18'}H_3$+$C^{18}H_3$; $CH_3$ du butyle; $CH_3$ isopropyl).

Exemple 5

N-(désacétyl-O-4 vinblastinoyl-23)-L-leucinate d'octyle (VL-octyl)

En appliquant le mode opératoire général on obtient la VL-octyl avec un rendement de 48%.

Propriétés physico-chimiques de la VL-Octyl

Point de fusion: ~ 145°C

$[\alpha]D^{CHCl_3}_{c=0,33}$: 54°

Spectre ultraviolet ($CH_3OH$, $\lambda$ max, nm, log $\varepsilon$):

8,62 mg/l

217 (4,69); 265 (4,14); 289 (4,01); 295 (3,95).

Spectre infra-rouge (KBr, $cm^{-1}$):

3460; 2940; 2920; 2870; 1735; 1665; 1610; 1500; 1455.

Spectre de résonance magnétique nucléaire ($CDCl_3$, ppm, 60MHz):

9,6 (1H,s,$C^{16}$-OH); 8,1 (1H,s,N'-H); 7,55 (1H,m); 7,23 (3H,m); 6,5 (1H,s,$C^9$-H); 6,12 (1H,s,$C^{12}$-H); 5,90 (2H,m,$C^{14}$-H + $C^{15}$-H); 4,2 (2H,t,—O—$CH_2$—Oct; 1H,d,$C^{17}$-H); 3,83 (3H,s,—$COOCH_3$); 3,67 (3H,s,—$OCH_3$); 3,56 (1H,s,$C^5$—H); 2,83 (3H,s,N—$CH_3$);

1,33 (10H,m, massif octyl + $CH_2^{19'}$ + $CH_2^{19}$); 1 (15H,m,massif octyl + $CH_3^{18}$ + $CH_3^{18'}$).

Exemple 6

N-(désacétyl-O-4 vinblastinoyl-23)-D-leucinate d'éthyle (VDLE)

En appliquant le mode opératoire général on obtient la VDLE avec un rendement de 74%.

Propriétés physico-chimiques de la VDLE:
Point de fusion: ~ 181°C
$[\alpha]D^{CHCl_3}_{c=0,28}$: 70°

Spectre ultraviolet (méthanol, $\lambda$ max, nm, log $\varepsilon$):
220 (4,67); 226 (4,20); 288 (4,11); 295 (4,02).

Spectre infrarouge (KBr, $cm^{-1}$):
3460; 2960; 2880; 1735; 1665; 1605.

Spectre de mass (m/e %):
924 (14) $M^+$ +28; 910 (32); $M^+$ +14; 897 (38) $M^+$ +1; 896 (66) $M^+$; 863 (28); 835 (43); 709 (43); 651 (81); 570 (100).

Spectre de résonance magnétique nucléaire ($CDCl_3$, ppm, 60MHz):
9,6 (1H,s,$C^{16}$-OH); 8,16 (1H,s,$N'^{\alpha}$-H); 7,66 (1H,m); 7,26 (3H,m); 6,7 (1H,s,$C^9$-H); 6,16 (1H,s,$C^{12}$-H); 5,90 (2H,m,$C^{14}$-H + $C^{15}$-H); 4,26 (2H,q,—$COOCH_2$); 3,83 (3H,s,—$OCH_3$); 3,66 (3H,s,—$OCH_3$); 2,90 (3H,s,—$N^{\alpha}$-$CH_3$); 1,3 (3H,t,—($COOCH_2$—$CH_3$; 0,96 (12H,m,$C^{18'}$ $H_3$ + $C^{18}H_3$ + isopropyl).

Exemple 7

N-désacétyl-O-4 vinblastinoyl-23)-L-sérinate d'éthyle (VSE)

En appliquant le mode opératoire général on obtient la VSE avec un rendement de 35%.

Propriétés physico-chimiques de la VSE:
$[\alpha]D^{CHCl_3}_{c=0,7}$: 65°

Spectre ultraviolet (MeOH, $\lambda$ max, nm, log $\varepsilon$):
215 (4,76); 266 (4,31); 288 (4,20); 297 (4,15).

Spectre infrarouge ($CHCl_3$, $cm^{-1}$):
3460; 3400; 2965; 2935; 2880; 1730; 1665; 1605.

Spectre de résonance magnétique nucléaire ($CDCl_3$, ppm, 60MHz):
8,13 (1H,s,$N^{\alpha'}$-H); 7,9 (1H,d,—OH); 7,52 (1H,m); 7,20 (3H,m); 6,63 (1H,s,$C^9$-H); 6,1 (1H,s,$C^{12}$-H); 5,9 (2H,m,$C^{14}$-H + $C^{15}$-H); 4,3 (2H,q,—COO—$CH_2$—); 3,83 (3H,s,—$OCH_3$); 3,66 (3H,s,—$OCH_3$); 2,67 (3H,s,—$N^{\alpha}CH_3$); 1,34 (3H,t,(—COO—$CH_2$)$CH_3$); 0,95 (6H,m,$C^{18'}H_3$ + $C^{18}H_3$).

Exemple 8

N-(désacétyl-O-4 vinblastinoyl-23)-L-glutamate d'éthyle (VGE)

En appliquant le mode opératoire général on obtient la VGE avec un rendement de 55%.

Propriétés physico-chimiques de la VGE:

Point de fusion: 149°C
$[\alpha]D^{CHCl_3}_{c=2}$: 59°

Spectre ultraviolet (MeOH, 10mg/1, $\lambda$ max, nm, log $\varepsilon$):
219 (4,46); 269 (3,94); 288 (3,80); 296 (3,76).

Spectre infra-rouge (KBr, $cm^{-1}$):
3460; 3395; 2960; 2920; 2870; 1730; 1665; 1610; 1500.

Spectre de résonance magnétique nucléaire ($CDCl_3$, ppm, 60MHz):
9,63 (1H,s,$C^{16}$-OH); 8,10 (1H,s,$N^{\alpha'}$-H); 7,6 (1H,m); 7,16 (3H,m); 6,64 (1H,s,$C^9$-H); 6,07 (1H,s,$C^{12}$-H); 5,83 (2H,m,$C^{14}H$+$C^{15}H$); 4,23 (2H,q,—$COOCH_2$—); 4,16 (2H, q, —$COOCH_2$—); 3,8 (3H,s,—$OCH_3$); 3,64 (3H,s,—$OCH_3$); 3,5 (1H,s,$C^5$—H); 2,8 (3H,s,—$N^{\alpha}CH_3$); 1,33 (3H,t,—$CH_3$); 1,26 (3H,t,—$CH_3$); 1 (6H,m,$C^{18}H_3$ + $C^{18'}H_3$).

Exemple 9

N-(désacétyl-O-4 vinblastinoyl-23)-L-phényl-alaninate d'éthyle (VPE)

En appliquant le mode opératoire général on obtient la VPE avec un rendement de 66%.

Propriétés physico-chimiques de la VPE:
Point de fusion: 154°C
$[\alpha]D^{CHCl_3}_{c=1,2}$: 78°

Spectre ultraviolet (MeOH, 9,8 mg/l, $\lambda$ max, nm, log $\varepsilon$):
217,5 (4,67); 265 (4,13); 286,5 (3,99); 295,5 (3,95).

Spectre infra-rouge (KBr, $cm^{-1}$):
3560, 3460; 3400; 2860; 2830; 1730; 1650; 1610; 1500; 1455.

Spectre de masse (m/e, %):
958 (17); 944 (41); 930 (35); 871 (64); 651 (41); 588 (41); 571 (53); 401 (100).

Spectre de résonance magnétique nucléaire ($CDCl_3$, ppm, 360MHz);
9,48 (1H,bs,—$C^{16}$—OH); 8,1 (1H,s,$N^{\alpha'}$-H); 7,6 (1H,d); 7,5 (1H,d); 7,3 à 7,02 (7H,m); 6,6 (1H,s,$C^9$-H); 6,1 (1H,s,$C^{12}$-H); 5,87 (2H,m,$C^{14}$-H + $C^{15}$-H) ($J^{15-14}$=12Hz; $J^{14-3}$=8,6Hz);
4,9 (1H,m

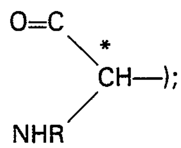

4,16 (2H,q,COO—$CH_2$); 3,8 (3H,s,—$OCH_3$); 3,56 (3H,s,—$OCH_3$); 3,43 (1H,s,$C^5$—H); 2,74 (3H,s,$N^\alpha$-$CH_3$); 1,20 (3H,t,—$CH_3$ (ester)); 0,97 (3H,t,—$C^{18'}H_3$); 0,88 (3H,t,—$C^{18'}H_3$).

Exemple 10

N-désacétyl-O-4 vinblastinoyl-23)-L-isoleucinate de méthyle (VILM)

En appliquant le mode opératoire général on obtient la VILM avec un rendement de 66%.

Propriétés physico-chimiques de la VILM
$[\alpha]D^{CHCl_3}_{C=0,135}$: ~ 66°

Spectre ultraviolet (MeOH, $\lambda$ max, nm, log $\varepsilon$):
225 (4,55); 266 (4,18); 288 (4,05); 295 (4,00).

Spectre de résonance magnétique nucléaire ($CDCl_3$, 360MHz):
9,48 (bs,1H,$C^{16}$-OH); 8,03 (s,1H,NH); 7,51 (M,2H); 7,23-7,06 (m,2H); 6,58 (s,1H,$C^9$-H); 6,20 (s,1H,$C^{12}$-H); 5,85 (dd,1H,$C^{14}$-H; $J^{15-14}$=12Hz; $J^{14-3}$=3,6Hz); 5,78 (d,1H,$C^{15}$-H); 4,62 (m,1H

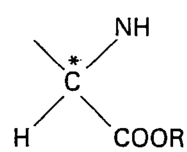
);

4,17 (d,1H); 3,77 (s,3H,$COOCH_3$); 3,75 (s,3H,$COOCH_3$); 3,6 (s,3H,—OMe—); 2,73 (s,3H,$N^\alpha$—$CH_3$); 2,58 (s,1H,$C^{21}$—H); 0,92 (m,12H,$C^{18}H_3$—$C^{18'}$H).

Exemple 11

N-(désacétyl-O-4 vinblastinoyl-23)-L-tyrosinate d'éthyle (V-Tyr E)

En appliquant le mode opératoire général on obtient la V-Tyr E avec un rendement de 48%.

Propriétés physico-chimiques de la V-Tyr E
$[\alpha]D^{CHCl_3}_{c=0,1087}$: 64°

Spectre ultra-violet ($CH_3OH$, $\lambda$ max, nm, log $\varepsilon$):
227 (4,73); 266 (4,26); 288 (4,07); 296 (3,94).

Spectre infra-rouge (KBr, $cm^{-1}$):
3460, 3400, 3040, 2950, 2840, 1715, 1660, 1610, 1500, 1455, 1225.

Spectre de résonance magnétique nucléaire (360 MHz):
9,6 (bs,1H,$C^{16}$-OH); 8,05 (s,1H,NH); 7,55 (m,2H); 7,21-7,06 (m,2H); 7,03 (d,2H,arom tyr J=7,5);

6,7 (d,2H,arom tyr.J=7,5); 6,55 (s,1H,$C^9$-H); 6,05 (s,1H,$C^{12}$-H); 5,83 (dd,1H,$C^{14}$-H; $J^{15-14}$=12Hz; $J^{14-3}$=3,6 Hz); 5,76 (d,1H, $C^{15}$—H); 4,83 (m,1H,CH); 4,13 (massif 3H,—$COOCH_2$,$C^{17}$—H); 3,76 (s,3H,—$OCH_3$—); 3,6 (s,3H,—$COOCH_3$); 3,45 (s,1H,$C^5$—H); 2,71 (s,3H,—$N^{\alpha}$—$CH_3$); 1,21 (t,3H,—$CH_3$—($CH_2OOC$)); 0,88 (m,6H,—$C^{18}H_3$—$C^{18'}H_3$).

### Exemple 12

N-(désacétyl-O-4 vinblastinoyl-23)-L-tryptophanate d'éthyle (V-Trypt E)

En appliquant le mode opératoire général on obtient la V-Trypt E avec un rendement de 72%.

Propriétés physico-chimiques de la V-Trypt E

$[\alpha]D\,^{CHCl_3}_{c\,=\,0,51}$: ~ 90°

Spectre ultra-violet (MeOH, $\lambda$ max, nm, log $\varepsilon$):

225 (5,15); 267 (4,65); 280 (ep); 290 (4,55).

Spectre de masse (m/e):

970, 391, 279, 165, 108, 35; ionisation moléculaire à l'isobutane 998 $M^+$ + 29, 984 $M^+$ + 15, 970 $M^+$ + 1, 926

Spectre infra-rouge (KBr, $cm^{-1}$)

3460, 3400, 3040, 2960, 2940, 2880, 1725, 1660, 1610, 1500, 1455, 1225, 740

Spectre de résonance magnétique nucléaire ($CDCl_3$, 360MHz):

9,5 (1H,s,$C^{16}$-OH); 8,2 (1H,s,NH,trypt); 8,03 (1H,s,NH); 7,66 (1H,d,trypt); J = 7,2Hz); 7,58 (1H,d,trypt; J = 7,2Hz); 7,51 (1H,d,trypt; J = 7, 2Hz); 7,31 (1H,d,trypt; J = 7,2Hz); 7,25—7,04 (5H,m); 6,58 (1H,s,$C^9$-H); 6,06 (1H,s,$C^{12}$-H); 5,83 (1H,dd,$C^{14}$-H; J = 12Hz; J = 3,6Hz); 5,78 (1H,d,$C^{15}$-H; J = 12Hz); 4,95 (1H,q,

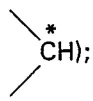

);

3,75 (3H,s,—$COOCH_3$); 3,6 (3H,s,—$OCH_3$); 3,4 (1H,s,$C^5$-H); 2,77 (1H,s,N—$CH_3$); 1,15 (3H,t,—$COOCH_2(CH_3)$).

### Exemple 13

N-(désacétyl-O-4 vinblastinoyl-23)-L-tryptophanate de méthyle (V-Trypt M)

En appliquant le mode opératoire général on obtient le V-Trypt M avec un rendement de 41%.

Propriétés physico-chimiques de la V-Trypt M:

$[\alpha]D\,^{CHCl_3}_{c\,=\,1,82}$: ~ 94°

Spectre ultra-violet (MeOH, $\lambda$ max, nm, log $\varepsilon$):

269 (4,48); 280; 289 (4,32).

Spectrre infra-rouge (KBr, $cm^{-1}$):

1730, 1710, 1660, 1610, 1495, 1455, 1220, 740.

Spectre de résonance magnétique nucléaire (360 MHz):

9,5 (1H,bs,$C^{16}$-OH); 8,06 (1H,s,NH ind); 8,01 (1H,s,NH ind); 7.65 (1H,d); 7,58 (1H,d); 7,38—7,06 (7H,m); 6,41 (1H,s,$C^9$-H); 6,05 (1H,s,$C^{12}$-H); 5,85 (1H,dd,$C^{14}$-H; J=12Hz; J'=3,6Hz); 5,78 (1H,d,$C^{15}$-H; J=12Hz); 4,95 (1H,q,C); 4,2 (1H,m,$C^{17-H}$); 3,75 (3H,s,—$OCH_3$); 3,61 (3H,s,—$COOCH_3$); 3,58 (3H,s,—$COOCH_3$); 3,43 (2H,s,$C^5$—H); 2,6 (3H,s,N—$CH_3$).

### Exemple 14

N-(désacétyl-O-4 vinblastinoyl-23)-L-valinate d'éthyle (VVE)

En appliquant le mode opératoire général on obtient la VVE avec un rendement de 63%.

Propriétés physico-chimiques de la VVE

Spectre ultra-violet (MeOH, $\lambda$ max, nm, log $\varepsilon$):

226 (4,95); 267 (4,57); 285 (4,45); 296 (4,40).

Spectre de masse (m/e %):
910 (0,1); 896 (2); 882 (5); 823 (4,5); 822 (6); 708 (5,3); 653 (9,2); 651 (8,7); 650 (14,6); 572 (8, 7); 571 (23,3); 539 (10,7); 355 (16); 354 (10); 353 (31); 294 (17); 188 (8,5); 156 (100); 155 (11,8); 154 (11,3); 144 (12,2); 141 (12,1); 140 (16,4); 136 (13); 135 (20,4); 124 (61); 122 (35,5); 121 (15,3).

Spectre infra-rouge (KBr, $cm^{-1}$):
3540, 3470, 3420, 2960, 2930, 2870, 1730, 1720, 1670, 1610, 1500, 1455, 1225, 745.

Spectre de résonance magnétique nucléaire:
9,48 (1H,bs,$C^{16}$-OH); 8,03 (1H,bs,NH ind); 7,55 (1H,d; J = 7,2Hz); 7,51 (1H,d,J = 7,2Hz); 7,51 (1H,d); 7,23 à 7,06 (3H,m); 6,58 (1H,s,$C^9$-H); 6,06 (1H,s,$C^{12}$-H); 5,85 (1H,dd,$C^{14}$-H; $J^{14-15}$ = 12Hz; $J^{14-3}$ = 3,6Hz); 5,78 (1H,d,$C^{15}$-H; J = 12 Hz); 4,56 (1H,dd,$\overset{*}{C}$); 4,21 (2H,q,$COOCH_2$); 4,15 (1H,d,$C^{17}$-H); 3,96 (1H,t); 3,76 (3H,s,$OCH_3$); 3,6 (3H,s,$COOCH_3$); 3,46 (1H,s,$C^5$-H); 2,73 (3H,s,N—$CH_3$); 1,31 (6H,m,$COOCH_2CH_3$); 0,96 (14H,m); 0,9 (14H,t).

Exemple 15

N-(désacétyl-O-4 vinblastinoyl-23)-L-iso-leucinate d'éthyle (VILE)

En appliquant le mode opératoire général on obtient la VILE avec un rendement de 58%.

Propriétés physico-chimiques de la VILE

Spectre ultra-violet (MeOH, $\lambda$ max, nm, log $\varepsilon$):
226 (4,94); 266 (4,56); 285 (4,44); 295 (4,38).

Spectre de masse (m/e %):
924 (7); 910 (11); 897 (23); 896 (44,4); 867 (11,3); 837 (18,7); 836 (26,6); 822 (4); 709 (10); 650 (21); 571 (13); 570 (34,7); 366 (21,7); 154 (100); 126 (11).

Spectra infra-rouge (KBr, $cm^{-1}$):
3460, 3440, 3040, 2960, 2940, 2880, 1730, 1665, 1610, 1500, 1455, 1225, 745.

Spectre de résonance magnétique nucléaire ($CDCl_3$, 360MHz):
9,46 (1H,bs,$C^{16}$-OH); 8,03 (1H,bs,NH); 7,55 (1H,d; J = 7,2Hz); 7,51 (1H,d); 7,23 à 7,06 (3H,m); 6,58 (1H,s,$C^9$-H); 6,06 (1H,s,$C^{12}$-H); 5,85 (1H,dd,$C^{14}$-H; J = 12Hz; J' = 3,6Hz); 5,78 (1H,d,$C^{15}$-H; J = 12Hz); 4,61 (1H,q,(dd), $\overset{*}{C}$); 4,21 (2H,q,COO—$CH_2$—); 4,15 (1H,d,$C^{17}$-H); 3,96 (1H,t); 3,76 (3H,s,$OCH_3$ ester); 3,6 (3H,s,$OCH_3$); 3,46 (1H,s,$C^5$—H); 2,73 (3H,s,N—$CH_3$); 1,25 (3H,t,$COOCH_2CH_3$)

Exemple 16

N-(désacétyl-O-4 vinblastinoyl-23)-D-tryptophanate d'éthyle (VD Trypt E)

En appliquant le mode opératoire général on obtient la VD Trypt E avec un rendement de 69%.

Propriétés physico-chimiques de la VD Trypt E
$[\alpha]D^{CHCl_3}_{c\,=\,0,3295}$: ~ 70°

Spectre ultra-violet (MeOH, $\lambda$ max, nm, log $\varepsilon$):
225 (4,77); 269 (4,30); 290 (4,20); 320 (ep).

Spectre de masse (m/e %):
970, 391, 279, 165, 108, 35.

Spectre infra-rouge (KBr, $cm^{-1}$):
3460, 3400, 3050, 2960, 2940, 2870, 1735, 1665, 1610, 1495, 1455, 1210, 740.

Spectre de résonance magnétique nucléaire:
9,53 (1H,bs,$C^{16}$-OH); 8,1 (1H,s,NH ind tryp); 8,02 (1H,s,NH ind); 7,76 (1H,d); 7,65 (1H,d); 7,51 (1H,d); 7,35 (1H,d); 7,2 à 7,05 (6H,m); 6,53 (1H,s,$C^9$H); 5,98 (1H,dd,$C^{14}$H $J^{14-15}$ = 12Hz, $J'^{14-3}$ = 3,6 Hz); 5,73 (1H,d,$C^{15}$-H; J = 12Hz); 4,86 (1H,q,C—); 4,1 (3H,m,$C^{17}$-H—$COOCH_2$); 3,75 (3H,s,$COOCH_3$); 3,6 (3H,s,—$OCH_3$); 3,33 (2H,s,$C^5$—H); 2,43 (3H,s,N—$CH_3$); 1,16 (3H,t,—$COOCH_2$—$CH_3$); 0,93 (8H,m).

Exemple 17

N-(désacétyl-O-4 vinblastinoyl-23)-L-valinyl-L-tryptophanate d'éthyle (V-Val-Trypt-E)

En appliquant le mode opératoire général on obtient la V-Val-Trypt E avec un rendement de 40%.

Propriétés physico-chimiques de la V-Val-Trypt-E
$[\alpha]D^{CHCl_3}_{c=0,3589}$: 36°

Spectre ultra-violet (méthanol, $\lambda$ max, nm, log $\varepsilon$):
270 (4,42); 290; 312 (4,96).

Spectre infra-rouge (KBr, $cm^{-1}$):
3480, 3400, 2960, 2940, 2870, 1735, 1725, 1660, 1610, 1500, 1455, 1230.

Spectre de résonance magnétique nucléaire ($CDCl_3$,360MHz):
9,5 (1H,bs,$C^{16}$-OH); 8,18 (1H,s,NH ind); 8,01 (1H,s,NH ind); 7,6 à 7,06 (10H,m arom); 6,41 (1H,s,$C^9$-H); 5,95 (1H,s,$C^{12}$-H); 5,85 (1H,dd,$C^{14}$-H); $J^{15-14}$ = 12Hz; $J^{8-14}$ = 3,6Hz) 5,75 (1H,d,$C^{15}$-H; $J^{15-14}$ = 12Hz); 5,02 (1H,m,C); 4,9 (1H,m,$\overset{*}{C}$); 3,73 (3H,s,$COOCH_3$); 3,56 (3H,s,—$OCH_3$); 3,43 (2H,s,$C^5$—H); 2,61 (3H,s,N—$CH_3$); 1,18 (3H,t,—$COOCH_2$—$CH_3$); 1,05—0,86 (± 16H,m,$CH_3$).

## Exemple 18

N-(désacétyl-O-4 vinblastinoyl-23)-L-leucyl-L-alanyl-L-leucyl-L-alaninate d'éthyle (V-Leu-Ala-Leu-Ala-E)

En appliquant le mode opératoire général et en préparant l'azoture à partir de 700 mg de monohydrazide, le couplage avec 377 mg de L-Leu-L-Ala-L-Leu-L-Alaninate d'éthyle à 4°C pendant 60 heures fournit 213 mg de V-Leu-Ala-Leu-Ala-E pure. La purification est effectuée par passage du produit brut d'abord sur une colonne de silice 60 Merck (230 mesh) en utilisant comme éluant un mélange éther-MeOH sat $NH_3$ (86:14), puis sur une colonne identique en utilisant comme éluant un mélange isopropanol-acétate d'éthyle-cyclohexane (40:20:40).

Propriétés physico-chimiques de la V-Leu-Ala-Leu-Ala-E
Spectre UV (méthanol, 5,74 mg/100 cc, log $\varepsilon$): 266 (4,24); 285—288 (4,12); 296 (4,07)
Spectre de masse (DCI à l'isobutane, ionisation moléculaire)
1152 ($M^+$ + 1), 1166 ($M^+$ + 1 + 14), 1134, 1108, 1094

Spectre IR (KBr): 3400, 2960, 1740, 1660, 1620, 1506, 1460, 1225, 745 $cm^{-1}$.

Spectre de résonance magnétique nucléaire ($CDCl_3$, 360 MHz, ppm)
0,91, 1,27 (t), 1,40 (2d), 1,67 (dxd), 2,61 (s), 2,72 (s), 3,60 (s, 3,75 (s), 4,19 (q), 5,74 (d), 5,83 (dxd), 6,03 (s), 6,59 (s), 6,88 (d), 7,00 (d), 7,39 (d), 7,49 (d), 7,53 (d), 8,02 (s), 9,57 (s).

## Exemple 19

N-(désacétyl-O-4 vinblastinoyl-23)-L-tryptophyl-L-tryptophanate d'éthyle (V-Tryp-Tryp-E).

En appliquant le mode opératoire général et en utilisant 1 g d'hydrazide et 543 mg de L-tryptophyl-L-tryptophanate d'éthyle, on isole 268 mg de V-Tryp-Tryp-E pure. La purification est effectuée par passage sur une colonne de silice en utilisant comme éluant d'abord un mélange éther-méthanol saturé en NH3 (92:8) puis un mélange identique en proportion 86:14.

Propriétés physico-chimiques
Spectre UV (MeOH): max 272 (4,38), 278 (4,37), 2,90 (4,31) (49,6 mg/l) min 248 (4,18), 288 (4,30)

Spectre de masse: ionisation moléculaire à l'isobutane
1184, 1170, 1156 ($M^+$ + 1), 1112, 1098, 624, 498, 165
Spectre IR (KBr): 3410, 2970, 2880, 1725, 1665, 1615, 1500, 1460, 1230 $cm^{-1}$

## Exemple 20

N-(désacétyl-O-4 vinblastinoyl-23)-L-tryptophylglycinate d'éthyle (V-Tryp-Gly-E)

La condensation de 596 mg d'hydrazide et de 226 mg de L-tryptophylglycinate d'éthyle (41h, 4°C) fournit 200 mg de V-Tryp-Gly-E, après purification sur colonne de silice en utilisant comme éluant un mélange éther-méthanol dont la proportion en méthanol sat. en $NH_3$ augmente de 4 à 14%.

Spectre de masse: ionisation moléculaire à l'isobutane
$M^+$ + 1 1027, 1042, 1055, 1013, 983, 969.

## Exemple 21

N-(désacétyl-O-4-vinblastinoyl-23)-L-tryptophane (V-Trypt Ac)

En appliquant le mode opératoire général mais en utilisant l'acide aminé non estérifié, on obtient la V-Trypt Ac avec un rendement de 67%.

Spectre UV (MeOH, $\lambda$ max, nm, log $\varepsilon$)
270 (4,29); 289 (4,18); 321 (ép)

Spectre IR (KBr, $cm^{-1}$):
3400, 2860, 2820, 2760, 1720, 1650, 1605, 1590, 1500, 1455, 1225, 780.

Exemple 22

N-(vinblastinoyl-23)-L-valinate d'éthyle

Le N-(désacétyl-O-4 vinblastinoyl-23)-L-valinate d'éthyle (100 mg) est traité par un mélange de pyridine (2,5 ml) et d'anhydride acétique (2,5 ml) sous atmosphère inerte et sous agitation pendant 24 heures. Après addition de méthanol et concentration sous vide, le résidu est repris par du dichlorométhane et lavé à l'eau sat. en NaCl. La phase organique est séchée sur $Na_2SO_4$ et concentrée sous vide. Le produit est purifié par chromatographie sur couche mince en utilisant comme éluant un mélange isopropanol/acétate d'éthyle/cyclohexane (40/20/40). On recueille ainsi 29 mg de N-(vinblastinoyl-23)-L-valinate d'éthyle.

Spectre de masse: ionisation moléculaire à l'isobutane:
$M^+ + 1$ 925, $M^+ + 1 + 14$ 939, $M^+ + 1 + 28$ 953, 924, 907, 893, 882, 881, 867, 866, 392, 279

Spectre IR (KBr, $cm^{-1}$) 3480, 3430, 2970, 1740, 1690, 1615,

Spectre UV (54,2 mg/l, $\lambda$ max, log $\varepsilon$): 263 (4,28), 288 (4,17), 296 (4,13).

Spectre RMN ($CDCl_3$ 360 MHz, ppm)
9,76 (1H,s), 8,06 (s), 7,52 (1H,d), 7,43 (1H,d), 7,20 et 7,03 (3H,m), 6,59 (s), 6,06 (1H,s), 5,85 (1H,dxd), 5,29 (1H,d), 4,57 (1H,dxd), 3,77 (3H,s), 3,61 (3H,s), 2,69 (3H,s), 2,02 (3H,s), 1,28 (3H, t), 0,97 (3H, d), 0,95 (3Hd), 0,90 (3H,t), 0,81 (3H,t).

**Revendications pour les Etats contractants: BE CH DE FR GB IT LI LU NL SE**

1. Dérivés de la vinblastine répondant à la formule générale suivante:

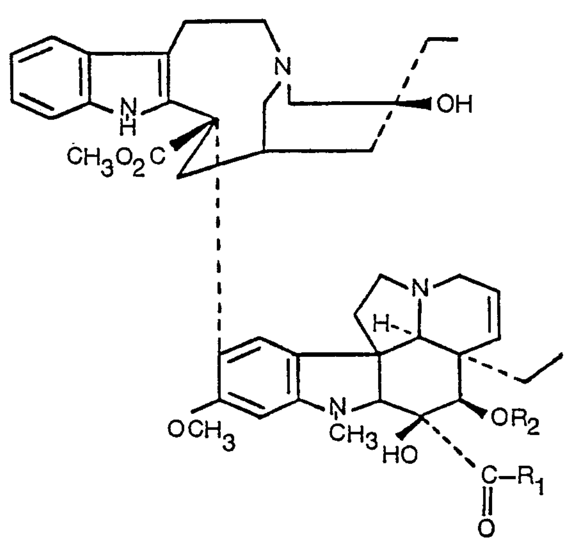

dans laquelle

$R_1$ représente un ester d'acide aminé ou de peptide.

couplé au fragment vinblastinoyl-23 par un lien amide, le peptide comprenant de 1 à 6 acides aminés différents ou identiques, et le groupe ester ramifié ou non possédant de 2 à 9 atomes de carbone; et

$R_2$ représente un atome d'hydrogène ou un groupe acyle comportant de 2 à 9 atomes de carbone, ainsi que ses sels d'addition avec les acides minéraux ou organiques.

2. Composés de formule générale

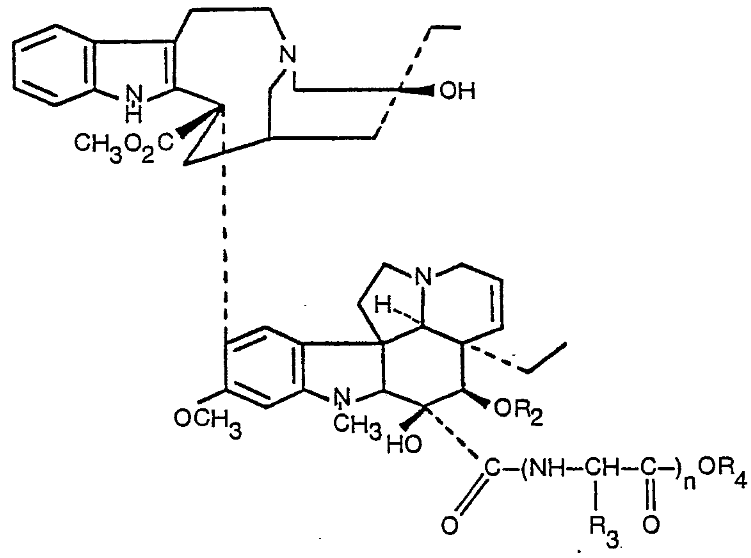

dans laquelle $R_2$ représente un atome d'hydrogène ou un groupe acyle comportant de 2 à 9 atomes de carbone, $R_3$ représente chaque fois indépendamment, un atome d'hydrogène, un groupe alkyle ramifié ou non comportant de 1 à 8 atomes de carbone, un groupe hydroxy C1-C8 alkyle, amino-hydroxy C1-C8 alkyle, carboxy-C1-C8 alkyle, amido C1-C8 alkyle, guanadino C1-C8 alkyle, amino C1-C8 alkyle, thiol C1-C8 alkyle cystéinyl méthyle, thiométhyl-éthyl, benzyle, hydroxybenzyle, I

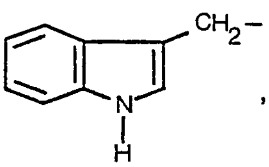 , 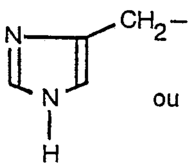 ou 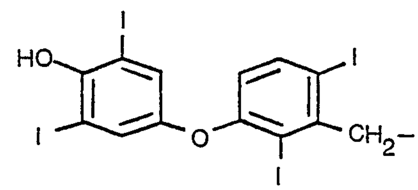

ou $R_3$, ensemble avec le carbone auquel il est rattaché et l'azote du groupe amide, forme un cycle azole ou hydroxyazole; n varie de 1 à 6 et $R_4$ est un groupe alkyle ramifié ou non comportant de 1 à 8 atomes de carbone, ou un groupe benzyle, ainsi que ses sels d'addition avec les acides minéraux ou organiques.

3. Composés suivant les revendications 1 et 2, caractérisés en ce qu'il s'agit du N-(désacétyl-O-4 vinblastinoyl-23)-L-tryptophanate d'éthyle ou de méthyle et leurs sels d'addition avec des acides pharmaceutiquement acceptables.

4. Composés suivant les revendications 1 et 2, caractérisés en ce qu'il s'agit du N-(désacétyl-O-4 vinblastinoyl-23)-isoleucinate d'éthyle ou de méthyle et leurs sels d'addition avec des acides pharmaceutiquement acceptables.

5. Composés suivant les revendications 1 et 2, caractérisés en ce qu'il s'agit du N-(désacétyl-O-4 vinblastinoyl-23)-L-valyl-L-tryptophanate d'éthyle et les sels d'addition avec des acides pharmaceutiquement acceptables.

6. Composés suivant les revendications 1 à 5, caractérisés en ce qu'ils se présentent sous la forme de leurs sels d'addition 1:1 avec l'acide sulfurique.

7. Le sulfate de N-(désacétyl-O-4 vinblastinoyl-23)-L-tryptophanate d'éthyle.

8. Composition pharmaceutique utilisée en médecine humaine et vétérinaire pour le traitement de maladies néoplastiques, caractérisée en ce qu'elle contient un ou plusieurs dérivés définis dans l'une quelconque des revendications 1 à 7.

9. Composition pharmaceutique selon la revendication 8, caractérisée en ce qu'elle est sous une forme d'administration dans laquelle le principe actif est présent à une dose unitaire comprise entre environ 2 et 900 mg.

10. Composition pharmaceutique selon la revendication 8, caractérisée en ce que le principe actif est le N-(désacétyl-O-4 vinblastinoyl-23)-L-tryptophanate d'éthyle, éventuellement sous la forme d'un sel d'addition avec un acide pharmaceutiquement acceptable.

11. Composition pharmaceutique selon la revendication 8, caractérisée en ce que le principe actif est le N-(désacétyl-O-4 vinblastinoyl-23)-L-valyl-L-tryptophanate d'éthyle, éventuellement sous la forme d'un sel d'addition avec un acide pharmaceutiquement acceptable.

12. Composition pharmaceutique selon la revendication 8, caractérisée en ce que, dans la forme d'administration parentérale, le principe actif se trouve dans un diluant pharmaceutiquement acceptable.

13. Composition pharmaceutique selon la revendication 12, caractérisée en ce que le diluant est une solution aqueuse stérile tamponnée.

14. Procédé d'obtention des composés selon les revendications 1 à 7, caractérisé en ce qu'il comprend les étapes suivantes:

1) réaction de la vinblastine avec l'hydrazine anhydre pour obtenir la désacétyl-O-4 carboxhydrazide-3 vinblastine,

2) traitement de l'hydrazide obtenu avec un agent de nitrosation en milieu acide pour obtenir l'azoture d'acide correspondant,

3) réaction de l'azoture d'acide avec 1 à 5 équivalents de l'ester d'acide aminé ou de peptide correspondant.

15. Procédé de préparation des composés selon les revendications 1 à 7, caractérisé en ce que l'azoture d'acide O-4-désacétyl vinblastinoïque-23 est traité par 1 à 5 équivalents d'ester d'acide aminé ou de peptide approprié, ledit peptide comportant de deux à six acides aminés.

16. Procédé de préparation du N-(désacétyl-O-4-vinblastinoyl-23)-L-tryptophanate d'éthyle, caractérisé en ce que la O-4-désacétyl-carboxhydrazide-3 déscarbométhoxy-3 vinblastine est traitée par un agent de nitrosation en milieu acide et l'azoture ainsi obtenu traité par le L-tryptophanate d'éthyle.

17. Procédé de préparation du N-(désacétyl-O-4 vinblastinoyl-23)-L-tryptophanate d'éthyle, caractérisé en ce que l'azoture d'acide O-4-désacétyl vinblastinoïque-23 est traité par 1 à 5 équivalents de L-tryptophanate d'éthyle.

18. Procédé selon la revendication 14, caractérisé en ce que l'agent de nitrosation est le nitrite de sodium en milieu acide chlorhydrique-méthanol.

19. Procédé selon la revendication 16, caractérisé en ce que l'agent de nitrosation est le nitrite de sodium en milieu acide chlorhydrique - méthanol.

20. Procédé selon la revendication 14, caractérisé en ce que le couplage avec l'ester d'acide aminé ou de peptide est effectué à une température comprise entre —3 et +10°C pendant 8 à 72 h.

**Revendications pour l'Etat contractant: AT**

1. Procédé d'obtention de dérivés de la vinblastine répondant à la formule générale suivante:

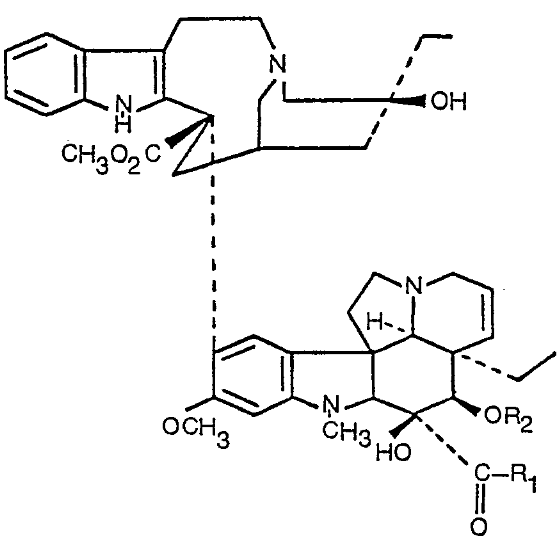

dans laquelle

$R_1$ représente un ester d'acide aminé ou de peptide, couplé au fragment vinblastinoyl-23 par un lien amide, le peptide comprenant de 1 à 6 acides aminés différents ou identiques, et le groupe ester ramifié ou non possédant de 2 à 9 atomes de carbone; et

$R_2$ représente un atome d'hydrogène ou un groupe acyle comportant de 2 à 9 atomes de carbone, ainsi que ses sels d'addition avec les acides minéraux ou organiques caractérisé en ce qu'il comprend les étapes suivantes:

1) réaction de la vinblastine avec l'hydrazine anhydre pour obtenir la désacétyl-O-4 carboxhydrazine-3 vinblastine,

2) traitement de l'hydrazide obtenu avec un agent de nitrosation en milieu acide pour obtenir l'azoture d'acide correspondant,

3) réaction de l'azoture d'acide avec 1 à 5 équivalents de l'ester d'acide aminé ou de peptide correspondant.

2. Procédé d'obtention de dérivés de la vinblastine répondant à la formule générale suivante:

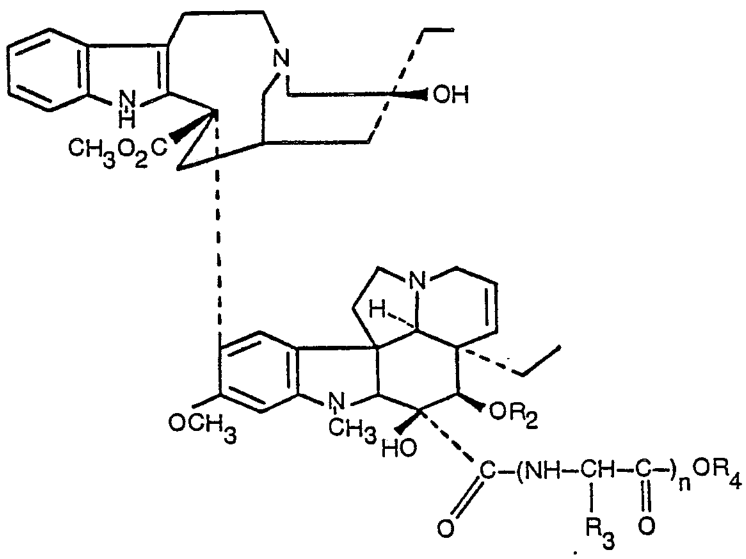

dans laquelle $R_2$ représente un atome d'hydrogène ou un groupe acyle comportant de 2 à 9 atomes de carbone, $R_3$ représente chaque fois indépendamment, un atome d'hydrogène, un groupe alkyle ramifié ou non comportant de 1 à 8 atomes de carbone, un groupe hydroxy C1-C8 alkyle, amino-hydroxy C1-C8 alkyle, carboxy-C1-C8 alkyle, amido C1-C8 alkyle, guanidino C1-C8 alkyle, amino C1-C8 alkyle, thiol C1-C8 alkyle cystéinyl méthyle, thiométhyl-éthyl, benzyle, hydroxybenzyle, l

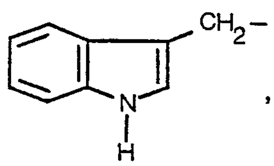

,

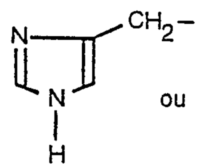

ou

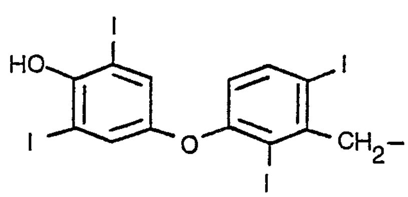

ou $R_3$ ensemble avec le carbone auquel il est rattaché et l'azote du groupe amide, forme un cycle azole ou hydroxyazole; n varie de 1 à 6 et $R_4$ est un groupe alkyle ramifié ou non comportant de 1 à 8 atomes de carbone, ou un groupe benzyle, ainsi que ses sels d'addition avec les acides minéraux ou organiques.

3. Procédé de préparation selon les revendications 1 et 2, caractérisé en ce que l'azoture d'acide O-4-désacétyl vinblastinoïque-23 est traité par 1 à 5 équivalents d'ester d'acide aminé ou de peptide approprié, ledit peptide comportant de deux à six acides aminés.

4. Procédé de préparation du N-(désacétyl-O-4-vinblastinoyl-23)-L-tryptophanate d'éthyle, caractérisé en ce que la O-4-désacétyl-carboxhydrazide-3 déscarbométhoxy-3 vinblastine est traitée par un agent de nitrosation en milieu acide et l'azoture ainsi obtenu traité par le L-tryptophanate d'éthyle.

5. Procédé de préparation du N-(désacétyl-O-4 vinblastinoyl-23)-L-tryptophanate d'éthyle, caractérisé en ce que l'azoture d'acide O-4-désacétyl vinblastinoïque-23 est traité par 1 à 5 équivalents de L-tryptophanate d'éthyle.

6. Procédé selon les revendications 1 et 2 caractérisé en ce que l'agent de nitrosation est le nitrite de sodium en milieu acide chlorhydrique — méthanol.

7. Procédé selon la revendication 4 caractérisé en ce que l'agent de nitrosation est le nitrite de sodium en milieu acide chlorhydrique - méthanol.

8. Procédé selon les revendications 1 à 7 caractérisé en ce que le couplage avec l'ester d'acide aminé ou de peptide est effectué à une température comprise entre —3° et +10°C pendant 8 à 72 h.

**Patentansprüche für die Vertragsstaaten: BE CH DE FR GB IT LI LU NL SE**

1. Vinblastinderivate der allgemeinen Formel

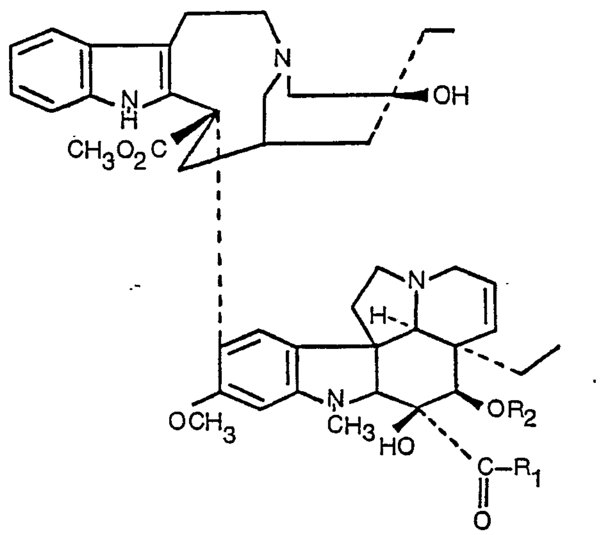

worin

$R_1$ einen Ester einer Aminosäure oder eines Peptids angebracht an den Vinblastin-23-oyl-Teil durch eine Amidbindung, darstellt, wobei das Peptid 1 bis 6 identische oder verschiedene Aminosäuren enthält und die verzweigte oder nicht verzweigte Estergruppe 2 bis 9 Kohlenstoffatome besitzt; und

$R_2$ ein Wasserstoffatom oder eine Acylgruppe mit 2—9 Kohlenstoffatomen ist, sowie deren Additionssalze mit Mineral- oder organischen Säuren.

2. Verbindungen der allgemeinen Formel

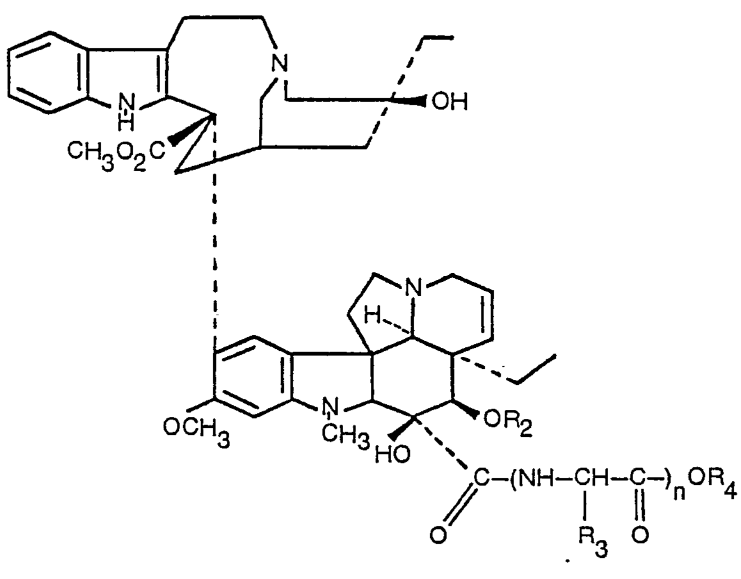

worin

$R_2$ ein Wasserstoffatom oder eine Acylgruppe mit 2—9 Kohlenstoffatomen darstellt;

$R_3$ jeweils unabhängig ein Wasserstoffatom, eine verzweigte oder nicht verzweigte Alkylgruppe mit 1—8 Kohlenstoffatomen, eine Hydroxy-$C_1$—$C_8$-alkyl-, Aminohydroxy-$C_1$—$C_8$-alkyl-, Carboxy-$C_1$—$C_8$-alkyl-, Amido-$C_1$-$C_8$-alkyl-, Guanadino-$C_1$-$C_8$-alkyl-, Amino-$C_1$—$C_8$-alkyl-, Thiol-$C_1$—$C_8$-alkyl-, Cysteinyl-methyl-, Thiomethyl-äthyl-, Benzyl- oder Hydroxybenzylgruppe,

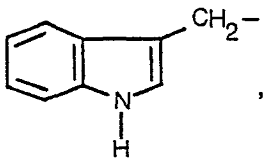 , 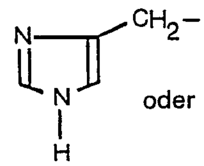 oder 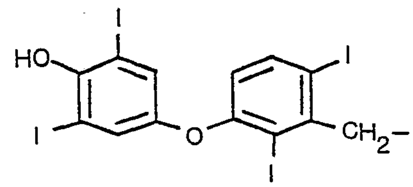

darstellt oder $R_3$ zusammen mit dem Kohlenstoffatom, an das es gebunden ist, und dem Stickstoffatom der Amidogruppe einen Azol- oder Hydroxyazolring bildet;

n zwischen 1 und 6 variiert; und

$R_4$ eine verzweigte oder nicht verzweigte Alkylgruppe mit 1—8 Kohlenstoffatomen oder eine Benzylgruppe ist, sowie deren Additionssalze mit Mineral- oder organischen Säuren.

3. Verbindungen nach den Ansprüchen 1 und 2, dadurch gekennzeichnet, dass es sich um Äthyl- oder Methyl N-(O-4-deacetyl-vinblastin-23-oyl)-L-tryptophanate und seine Additionssalze mit pharmazeutisch verträglichen Säuren handelt.

4. Verbindungen nach den Ansprüchen 1 und 2, dadurch gekennzeichnet, dass es sich um Äthyl- oder Methyl N-(O-4-deacetyl-vinblastin-23-oyl)-L-isoleucinat und seine Additionssalze mit pharmazeutisch verträglichen Säuren handelt.

5. Verbindungen nach den Ansprüchen 1 und 2, dadurch gekennzeichnet, dass es sich um Äthyl (deacetyl-O-4-vinblastin-23-oyl)-L-valyl-L-tryptophanat und seine Additionssalze mit pharmazeutisch verträglichen Säuren handelt.

6. Verbindungen nach den Ansprüchen 1—5, dadurch gekennzeichnet, dass sie in der Form ihrer 1:1-Additionssalze mit Schwefelsäure bestehen.

7. Äthyl N-(O-4-deacetyl-vinblastin-23-oyl)-L-tryptophanatsulfat.

8. Pharmazeutische Zusammensetzung zur Verwendung in der Human- und Veterinärmedizin zur Behandlung von neoplasmischen Krankheiten, dadurch gekennzeichnet, dass sie eine oder mehrere Verbindungen gemäss einem der Ansprüche 1 bis 7 enthalten.

9. Pharmazeutische Zusammensetzung nach Anspruch 8, dadurch gekennzeichnet, das sie aus einer Form der Verabreichung besteht, in der die aktive Verbindung in einer einheitlichen Dosis zwischen etwa 2 und 900 mg enthalten ist.

10. Pharmazeutische Zusammensetzung nach Anspruch 8, dadurch gekennzeichnet, dass die aktive Verbindung Äthyl N-(O-4-deacetyl-vinblastin-23-oyl)-L-tryptophanat, gegebenenfalls in der Form eines Additionssalzes mit einer pharmazeutisch verträglichen Säure ist.

11. Pharmazeutische Zusammensetzung nach Anspruch 8, dadurch gekennzeichnet, dass die aktive Verbindung Äthyl N-(O-4-deacetyl-vinblastin-23-oyl)-L-valyl-L-tryptophanat, gegebenenfalls in der Form eines Additionssalzes mit einer pharmazeutisch verträglichen Säure ist.

12. Pharmazeutische Zusammensetzung nach Anspruch 8, dadurch gekennzeichnet, dass, in der Form der parenteralen Verabreichung, die aktive Verbindung in einem pharmazeutisch verträglichen Verdünnungsmittel enthalten ist.

13. Pharmazeutische Zusammensetzung nach Anspruch 12, dadurch gekennzeichnet, dass das Verdünnungsmittel eine sterile gepufferte wässrige Lösung ist.

14. Verfahren zur Herstellung der Verbindung gemäss den Ansprüchen 1 bis 7, gekennzeichnet durch die folgenden Stufen:

1) Reaktion von Vinblastin mit wasserfreiem Hydrazin zum Erhalt des O-4-deacetyl-vinblastin-3-carboxhydrazids;

2) Behandlung des erhaltenen Hydrazids mit einem Nitrosierungsmittel in saurem Medium zum Erhalt des entsprechenden Säureazids;

3) Reaktion des Säureazids mit 1 bis 5 Äquivalenten des entsprechenden Aminosäure- oder Peptidesters.

15. Verfahren zur Herstellung der Verbindungen gemäss den Ansprüchen 1 bis 7, dadurch gekennzeichnet, dass das Azid der O-4-deacetyl-vinblastin-23-säure mit 1 bis 5 Äquivalenten des geeigneten Aminosäure- oder Peptidesters umgesetzt wird, wobei das Peptid 2 bis 6 Aminosäuren umfasst.

16. Verfahren zur Herstellung von Äthyl N-(O-4-deacetyl-vinblastin-23-oyl-)-L-tryptophanat, dadurch gekennzeichnet, dass O-4-deacetyl-3-decarbomethoxy-vinblastin-3-carboxyhydrazid mit einem Nitrosierungsmittel in saurem Medium umgesetzt wird, und das so erhaltene Azid mit Äthyl L-tryptophanat umgesetzt wird.

17. Verfahren zur Herstellung von Äthyl N-(O-4-deacetyl-vinblastin-23-oyl)-L-tryptophanat, dadurch gekennzeichnet, dass das Azid der O-4-deacetyl-vinblastin-23-Säure mit 1 bis 5 Äquivalenten von Äthyl L-tryptophanat umgesetzt wird.

18. Verfahren nach Anspruch 14, dadurach gekennzeichnet, dass das Nitrosierungsmittel Natriumnitrit ist im Medium Chlorwasserstoffsäure-Methanol.

19. Verfaren nach Anspruch 16, dadurch gekennzeichnet, dass das Nitrosierungsmittel Natriumnitrit ist im Medium Chlorwasserstoffsäure-Methanol.

20. Verfahren nach Anspruch 14, dadurch gekennzeichnet, dass die Bindung mit dem Aminosäureester oder dem Peptidester bei einer Temperatur zwischen —3 und +10°C, während 8 bis 72 Stunden, vorgenommen wird.

**Patentansprüche fur den Vertragsstaat: AT**

1. Verfahren zur Herstellung der Vinblastinderivate der allgemeinen Formel

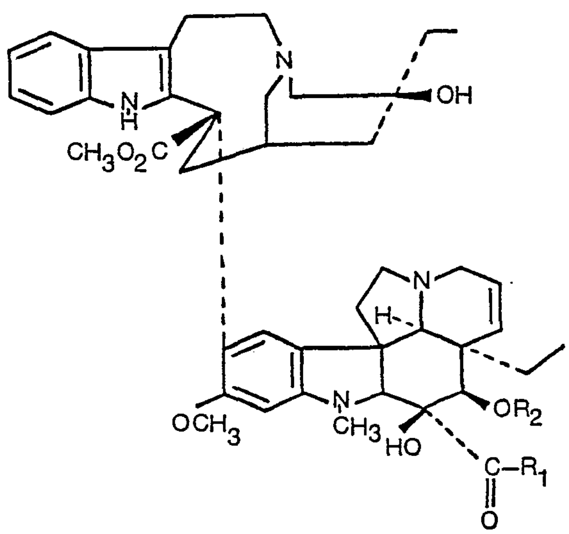

worin

$R_1$ einen Ester einer Aminosäure oder eines Peptids angebracht an den Vinblastin-23-oyl-Teil durch eine Amidbindung, darstellt, wobei das Peptid 1 bis 6 identische oder verschiedene Aminosäuren enthält und die verzweigte oder nicht verzweigte Estergruppe 2 bis 9 Kohlenstoffatome besitzt; und

$R_2$ ein Wasserstoffatom oder eine Acylgruppe mit 2—9 Kohlenstoffatomen ist, sowie ihre Additionssalze mit Mineral- oder organischen Säuren, gekennzeichnet durch die folgenden Stufen:

1) Reaktion von Vinblastin mit dem wasserfreien Hydrazin zur Herstellung des entsprechenden O-4-Deacetyl-vinblastin-3-carboxyhydrazids,

2) Behandlung des so erhaltenen Hydrazids mit einem nitrosierenden Mittel in saurem Medium zur Herstellung des entsprechenden Säureazids;

3) Reaktion des Säureazids mit 1 bis 5 Äquivalenten des Aminosäure- oder Peptidesters.

2. Verfahren zur Hestellung von Vinblastinderivaten der allgemeinen Formel

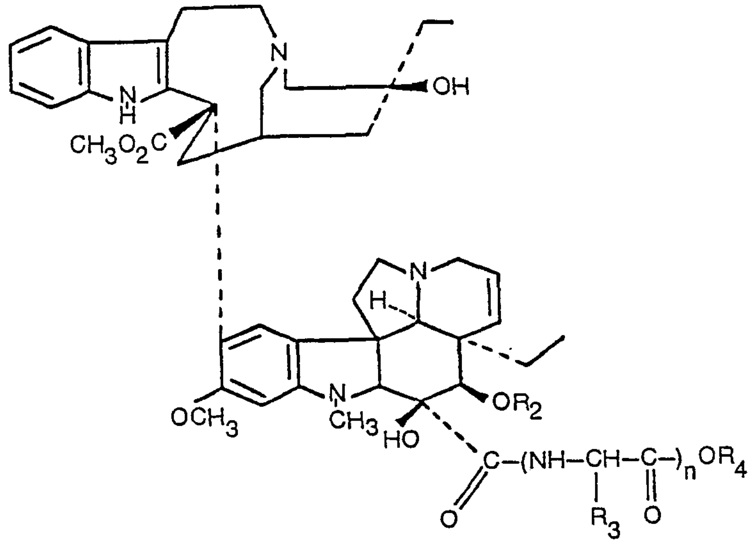

worin

$R_2$ ein Wasserstoffatom oder eine Acylgruppe mit 2—9 Kohlenstoffatomen darstellt;

$R_3$ jeweils unabhängig ein Wasserstoffatom, eine verzweigte oder nicht verzweigte Alkylgruppe mit 1 bis 8 Kohlenstoffatomen, eine Hydroxy-$C_1$—$C_8$-alkyl-, Amino-hydroxy-$C_1$—$C_8$-alkyl-, Carboxy-$C_1$—$C_8$- alkyl-, Amido-$C_1$—$C_8$-alkyl-, Guanadino-$C_1$—$C_8$-alkyl-, Amino-$C_1$—$C_8$-alkyl-, Thiol-$C_1$—$C_8$-alkyl-, Cysteinyl-methyl-, Thiomethyl-äthyl-, Benzyl- oder Hydroxybenzylgruppe,

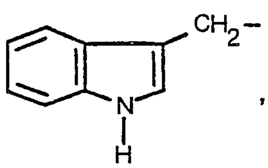 , 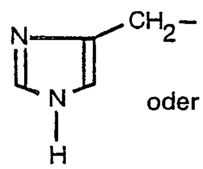 oder 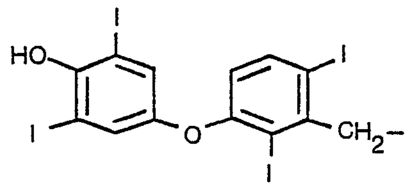

darstellt oder zusammen mit dem Kohlenstoffatom, an das es gebunden ist, und dem Stickstoffatom der Amidogruppe einen Azol- oder Hydroxyazolring bildet;

n zwischen 1 und 6 variiert; und

$R_4$ eine verzweigte oder nicht verzweigte Alkylgruppe mit 1 bis 8 Kohlenstoffatomen oder eine Benzylgruppe ist, sowie ihre Additionssalze mit Mineral- oder organischen Säuren.

3. Verfahren zur Herstellung der Verbindung gemäss Ansprüche 1 und 2, dadurch gekennzeichnet, dass das Azid der O-4-Deacetyl-vinblastin-23-säure mit 1 bis 5 Äquivalenten des geeigneten Aminosäure- oder Peptidesters umgesetzt wird, wobei das Peptid 2 bis 6 Aminosäuren enthält.

4. Verfahren zur Herstellung von Äthyl N-(O-4-deacetyl-vinblastin-23-oyl)-L-tryptophanat, dadurch gekennzeichnet dass O-4-Deacetyl-3-decarbomethoxy-vinblastin-3-carboxyhydrazid mit einem Nitrosiertungsmittel in saurem Medium umgesetzt wird, und das so erhaltene Azid mit Äthyl L-tryptophanat umgesetzt wird.

5. Verfahren zur Herstellung von Äthyl N-(O-4-deacetyl-vinblastin-23-oyl)-L-tryptophanat, dadurch gekennzeichnet, dass das Azid von O-4-Deacetyl-vinblastin-23-säure mit 1 bis 5 Äquivalenten L-tryptophanat umgesetzt wird.

6. Verfahren nach den Ansprüchen 1 und 2, dadurch gekennzeichnet, dass das Nitrosierungsmittel Natriumnitrit ist im Medium Chlorwasserstoffsäure-Methanol.

7. Verfahren nach Anspruch 4, dadurch gekennzeichnet, dass das Nitrosierungsmittel Natriumnitrit ist im Medium Chlorwasserstoffsäure-Methanol.

8. Verfahren nach den Ansprüchen 1 bis 7, dadurch gekennzeichnet, dass die Bindung mit dem Aminosäure-oder Peptidester bei einer Temperatur zwischen —3° und +10°C während bis 72 Stunden vorgenommen wird.

**Claims for the Contracting States: BE CH DE FR GB IT LI LU NL SE**

1. Vinblastine derivatives represented by the general formula:

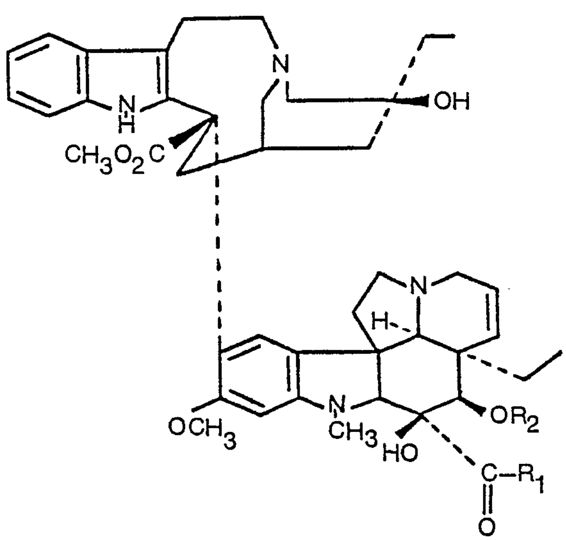

wherein

$R_1$ represents an amino-acid ester or a peptide ester, linked to the vinblastin-23-oyl portion by an amide

linkage, the peptide having from 1 to 6 identical or different amino-acids, and the ester group branched or not having from 2 to 9 carbon atoms; and

$R_2$ represents a hydrogen atom or an acyl group having from 2 to 9 carbon atoms, and their addition salts with mineral or organic acids.

2. Compounds of the general formula

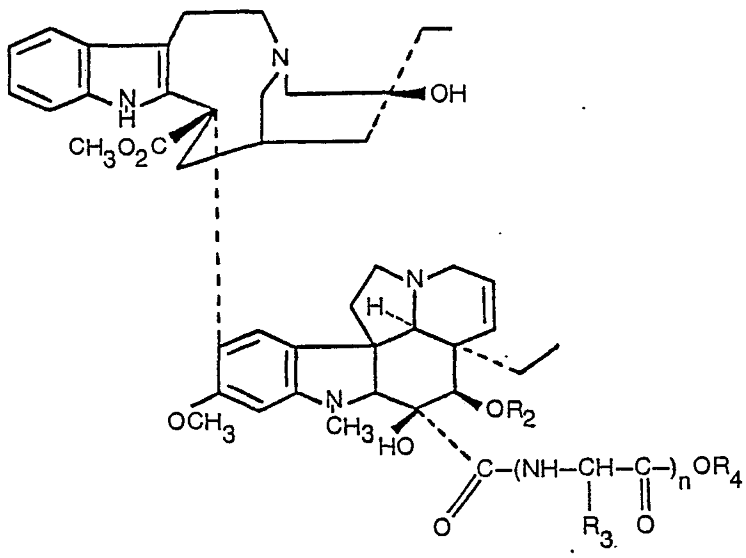

wherein $R_2$ is a hydrogen atom or an acyl group having from 2 to 9 carbon atoms, $R_3$ represents each time independently, a hydrogen atom, an alkyl group branched or not having from 1 to 8 carbon atoms, hydroxy C1—C8 alkyl group, amino-hydroxy C1—C8 alkyl, carboxy-C1—C8 alkyl, amido C1—C8 alkyl, guanadino C1—C8 alkyl, amino C1—C8 alkyl, thiol C1—C8 alkyl, cysteinylmethyl, thiomethyl-ethyl, benzyle, hydroxybenzyle group,

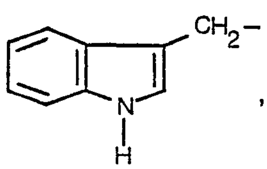 , 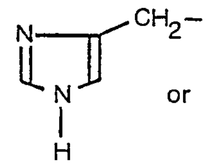 or 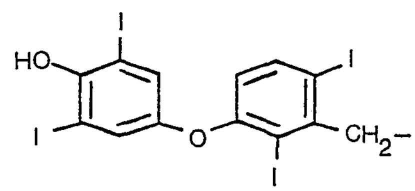

or $R_3$, together with the carbon to which it is attached and the amido nitrogen, forms an azole or hydroxyazole ring; n varies from 1 to 6 and $R_4$ is an alkyl group branched or not having from 1 to 8 carbon atoms, or a benzyl group, and their addition salts with mineral or organic acids.

3. Compounds according to claims 1 and 2, characterised in that it is ethyl or methyl N-(O-4-deacetyl-vinblastin-23-oyl)-L-tryptophanate and their pharmaceutically acceptable acid addition salts.

4. Compounds according to claims 1 and 2, characterised in that it is ethyl or methyl N-(O-4-deacetyl-vinblastin-23-oyl)-isoleucinate and their pharmaceutically acceptable acid addition salts.

5. Compounds according to claims 1 and 2, characterised in that it is ethyl N-(O-4-deacetyl-vinblastin-23-oyl)-L-valyl-L-tryptophanate and their pharmaceutically acceptable acid addition salts.

6. Compounds according to claims 1 to 5, characterised in that there are in the form of their 1:1 addition salts with sulfuric acid.

7. Ethyl N-(O-4-deacetyl-vinblastin-23-oyl)-L-tryptophanate sulfate.

8. A pharmaceutical composition used in human and veterenary medecine for the treatment of neoplastic deseases, characterised in that it comprises one or more derivatives defined in any of the claims 1 to 7.

9. A pharmaceutical composition according to claim 8, characterised in that it is in a form of administration wherein the active principle is present at an unitary dose varying between about 2 and 900 mg.

10. A pharmaceutical composition according to claim 8, characterised in that the active principle is ethyl N-(O-4-deacetyl-vinblastin-23-oyl)-L-tryptophanate, optionally in the form of an addition salt with a pharmaceutically acceptable acid.

11. A pharmaceutical composition according to claim 8, characterised in that the active principle is ethyl N-(O-4-deacetyl-vinblastin-23-oyl)-L-valyl-L-tryptophanate, optionally in the form of a pharmaceutically acceptable acid addition salt.

12. A pharmaceutical composition according to claim 8, characterised in that, in the parenteral adminstration form, the active principle is in a pharmaceutically acceptable diluent.

13. A pharmaceutical composition according to claim 12, characterised in that the diluent is a buffered sterile aqueous solution.

14. A process for the preparation of the compounds according to claims 1 to 7, characterised in that it comprises the following steps:

1- reacting vinblastine with anhydrous hydrazine to obtain O-4-deacetyl-vinblastin-3-carboxyamide,

2- treating the resulting hydrazide with a nitrosating reagent in an acid medium in order to obtain the corresponding acid azide,

3- reacting the acid azide with 1 to 5 equivalents of the corresponding aminoacid ester or peptide ester.

15. A process for the preparation of the compounds according to claims 1 to 7, characterised in that the azide of O-4-deacetyl-vinblastin-23-oic acid is treated by 1 to 5 equivalents of the appropriate aminoacid ester or peptide ester, said peptide comprising from 2 to 6 aminoacids.

16. A process for the preparation of ethyl N-(O-4-deacetyl-vinblastin-23-oyl)-L-tryptophanate, characterised in that the O-4 deacetyl-3-descarbomethoxy-vinblastin-3-carboxyhydrazide is treated with a nitrosating reagent in an acid medium and the resulting azide reacted with ethyl L-tryptophanate.

17. A process for the preparation of ethyl N-(O-4-deacetyl-vinblastin-23-oyl)-L-tryptophanate, characterised in that the azide of O-4-deacetyl-vinblastin-23-oic acid is treated with 1 to 5 equivalents of ethyl L-tryptophanate.

18. A process according to claim 14 characterised in that the nitrosating reagent is sodium nitrite in a hydrochloric acid—methanol medium.

19. A process according to claim 16, wherein the nitrosating reagent is sodium nitrite in a hydrochloric acid—methanol medium.

20. A process according to claim 14, wherein the coupling with the ester of aminoacid or peptide is effected at a temperature varying between −3 and +10°C for 8 to 72 h.

**Claims for the Contracting State: AT**

1. A process for the preparation of vinblastine derivatives of the general formula:

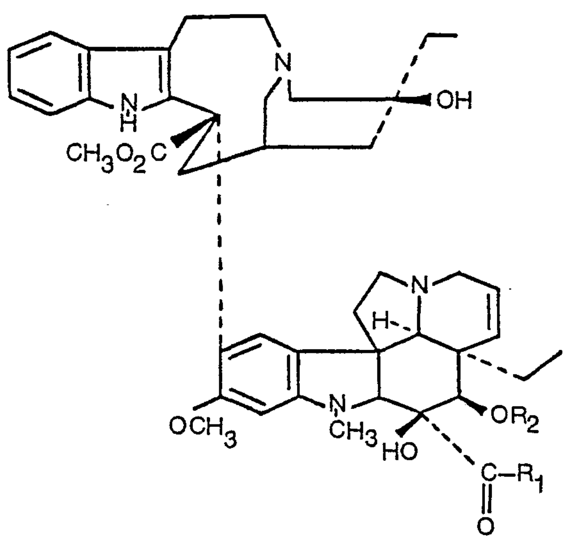

wherein

$R_1$ represents an amino-acid ester or a peptide ester, linked to the vinblastin-23-oyl portion by an amide bonding, the peptide having from 1 to 6 identical or different amino-acids, and the ester group branched or not having from 2 to 9 carbon atoms; and

$R_2$ represents a hydrogen atom or an acyl group having from 2 to 9 carbon atoms, and their addition salts with mineral or organic acids, characterised in that it comprises the following steps:

1- reacting vinblastine with anhydrous hydrazine to obtain O-4-deacetyl-vinblastin-3-carboxamide,

2- treating the resulting hydrazide with a nitrosating reagent in acid medium in order to obtain the corresponding acid azide,

3- reacting said acid azide with 1 to 5 equivalents of the corresponding aminoacid or peptide ester.

2. A process for the preparation of vinblastin derivatives of the general formula

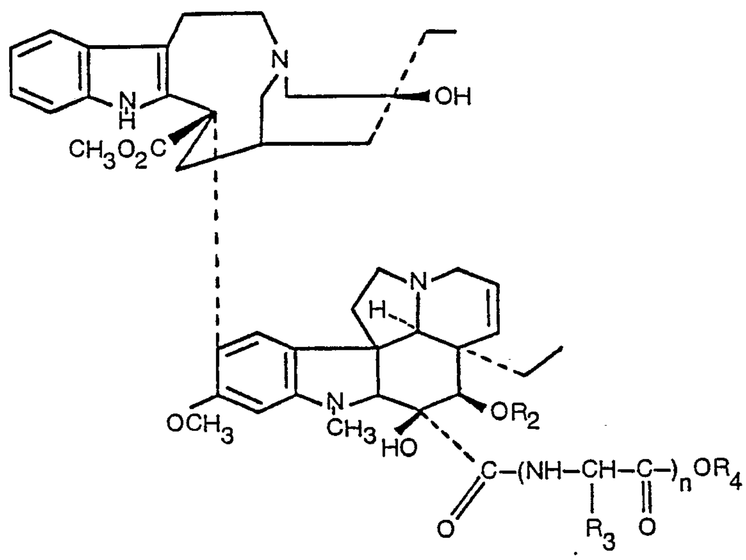

wherein $R_2$ is a hydrogen atom or an acyl group having from 2 to 9 carbon atoms, $R_3$ represents each time independently, a hydrogen atom, an alkyl group branched or not having from 1 to 8 carbon atoms, a hydroxy C1—C8 alkyl group, amino-hydroxy C1—C8 alkyl, carboxy-C1—C8 alkyl, amido C1—C8 alkyl, guanadino C1—C8 alkyl, amino C1—C8 alkyl, thiol C1—C8 alkyl, cysteinylmethyl, thiomethylethyl, benzyle, hydroxybenzyle group,

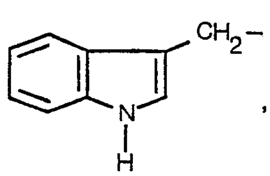 , 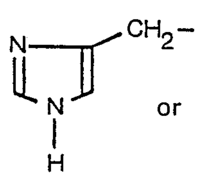 or 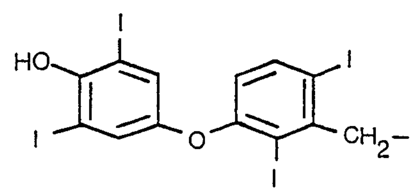

or $R_3$, together with the carbon to which it is attached and the amido nitrogen, forms an azole or hydroxyazole ring; n varies from 1 to 6 and $R_4$ is an alkyl group branched or not having from 1 to 8 carbon atoms, or a benzyl group, and their addition salts with mineral or organic acids.

3. A process for the preparation of the compounds according to claims 1 and 2, characterised in that the azide of O-4-deacetyl-vinblastin-23-oic acid is treated with 1 to 5 equivalents of the appropriate aminoacid ester or peptide ester, said peptide comprising from 2 to 6 aminoacides.

4. A process for the preparation of ethyl N-(O-4-deacetyl-vinblastin-23-oyl)-L-tryptophanate, characterised in that the O-4-deacetyl-3-descarbomethoxy-vinblastin-3-carboxhydrazide is treated with a nitrosating reagent in an acid medium and the resulting azide reacted with ethyl L-tryptophanate.

5. A process for the preparation of ethyl N-(O-4-deacetyl-vinblastin-23-oyl)-L-tryptophanate, characterised in that the azide of O-4-deacetyl-vinblastin-23-oic acid is treated with 1 to 5 equivalents of ethyl L-tryptophanate.

6. A process according to claim 1 and 2 characterised in that the nitrosating reagent is sodium nitrite in a hydrochloric acid—methanol medium.

7. A process according to claim 4, wherein the nitrosating reagent is sodium nitrite in a hydrochloric acid— methanol medium.

8. A process according to claim 4, wherein the coupling with the ester of the amino acid or peptide is effected at a temperature varying between —3° and +10°C for 8 to 72 hrs.